(19) Europäisches Patentamt
European Patent Office
Office européen des brevets

(11) EP 3 691 669 B1

(12) FASCICULE DE BREVET EUROPEEN

(45) Date de publication et mention de la délivrance du brevet:
**07.06.2023 Bulletin 2023/23**

(21) Numéro de dépôt: **18783439.5**

(22) Date de dépôt: **04.10.2018**

(51) Classification Internationale des Brevets (IPC):
***A61K 36/605*** $^{(2006.01)}$ ***A61P 17/02*** $^{(2006.01)}$
***A61K 8/9789*** $^{(2017.01)}$ ***A61Q 19/02*** $^{(2006.01)}$
***A61Q 19/08*** $^{(2006.01)}$

(52) Classification Coopérative des Brevets (CPC):
**A61K 36/605; A61K 8/9789; A61P 17/02; A61Q 19/02; A61Q 19/08**

(86) Numéro de dépôt international:
**PCT/EP2018/077042**

(87) Numéro de publication internationale:
**WO 2019/068824 (11.04.2019 Gazette 2019/15)**

# (54) EXTRAITS RACINAIRES DE PLANTES DU GENRE MORUS ET LEURS UTILISATIONS

WURZELEXTRAKTE AUS PFLANZEN DER MORUS-GATTUNG UND VERWENDUNGEN DAVON

ROOT EXTRACTS FROM PLANTS OF THE MORUS GENUS AND USES OF SAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.10.2017 FR 1759396**

(43) Date de publication de la demande:
**12.08.2020 Bulletin 2020/33**

(73) Titulaire: **Plant Advanced Technologies Pat**
**54500 Vandoeuvre-lès-Nancy (FR)**

(72) Inventeurs:
- **DURIOT, Léonor, Cécile**
  **54210 Saffais (FR)**
- **SALWINSKI, Aleksander, Bogusz**
  **54000 Nancy (FR)**
- **RANGONI, Léa, Iolé, Heidi**
  **67000 Strasbourg (FR)**

(74) Mandataire: **IPAZ**
**16, rue Gaillon**
**75002 Paris (FR)**

(56) Documents cités:
**EP-A1- 0 997 140** **WO-A1-2017/129779**
**WO-A2-2013/181296** **WO-A2-2015/149136**
**FR-A1- 2 616 328** **FR-A1- 2 868 308**

- **ZONG-PING ZHENG ET AL: "Tyrosinase Inhibitory Constituents from the Roots of Morus nigra : A Structure-Activity Relationship Study", JOURNAL OF AGRICULTURAL AND FOOD CHEMISTRY, vol. 58, no. 9, 12 mai 2010 (2010-05-12), pages 5368-5373, XP055479819, US ISSN: 0021-8561, DOI: 10.1021/jf1003607 cité dans la demande**
- **KANG KYO BIN ET AL: "Prediction of tyrosinase inhibitory activities ofMorus albaroot bark extracts from HPLC fingerprints", MICROCHEMICAL JOURNAL, vol. 110, 2013, pages 731-738, XP028737620, ISSN: 0026-265X, DOI: 10.1016/J.MICROC.2013.08.012 cité dans la demande**
- **ZONG-PING ZHENG ET AL: "Tyrosinase inhibition constituents from the roots of", FITOTERAPIA, vol. 83, no. 6, 2012, pages 1008-1013, XP028427381, ISSN: 0367-326X, DOI: 10.1016/J.FITOTE.2012.06.001 [extrait le 2012-06-11]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 691 669 B1

- Dr Sulochana ET AL: "Medicinal Values of Mulberry -An Overview", Journal of Pharmacy Research Journal of Pharmacy Research, 1 janvier 2012 (2012-01-01), pages 3588-3596, XP055479829, Extrait de l'Internet: URL:http://jprsolutions.info/newfiles/journal-file-56be002604fce2.03809922.pdf [extrait le 2018-05-30]

**Description**

**DOMAINE DE L'INVENTION**

La présente invention se situe dans le domaine des extraits végétaux et concerne un extrait racinaire de plantes du genre *Morus* (en particulier *Morus alba* et *Morus nigra)* riche en polyphénols prenylés, un procédé de préparation d'un tel extrait ainsi qu'une composition cosmétique et une composition pharmaceutique, lesdites compositions comprenant en tant qu'agent actif au moins un extrait racinaire de plantes du genre *Morus* selon l'invention.

**ART ANTERIEUR**

Les plantes du genre *Morus,* arbre ou arbuste, de la famille des Moracées peuvent être sources de composés phénoliques présentant des activités bénéfiques (The latest review on the polyphenols and their bioactivities of Chinese Morus plants ; Yang Y. et al ; J Asian Nat Prod Res. 2014; 16(6): 690-702). L'écorce de racines, les tiges et les feuilles des plantes du genre *Morus,* et en particulier du mûrier blanc, sont traditionnellement utilisées en médecine chinoise pour le traitement du diabète, de l'arthrose ou de rhumatisme. Parmi leurs nombreuses vertus, les extraits sont connus pour leur activité hypotensive, antiinflammatoire et antimicrobienne. Il a été démontré que ces propriétés étaient dues à leur contenu en polyphénols, notamment des dérivés de resvératrol tels que le mulberroside A, des adduits de type Diels-Alder, et des flavonoïdes prenylés de type moracénines ou kuwanones.

La tyrosinase est l'enzyme limitante du taux de synthèse de la mélanine et elle est la cible principale des remèdes contre l'hyperpigmentation. Des extraits issus d'écorces de racines, de tiges et de feuilles de *Morus alba, Morus nigra* et *Morus australis* sont utilisés couramment dans l'industrie cosmétique comme agent de blanchiment de la peau. Ces extraits présentent des composés inhibant l'activité de la tyrosinase, comme la moracénine A et la moracénine B identifiées dans les racines de *Morus nigra (*Tyrosinase inhibitory constituents from the roots of Morus nigra: a structure-activity relationship study ; Zheng ZP et al ; J Agric Food Chem. 2010 May 12;58(9):5368-73 ) ou dans l'écore de racine de *Morus alba (*Prédiction of tyrosinase inhibitory activities of Morus alba root bark extracts from HPLC fingerprints; Kyo Bin Kang et al; Microchemical Journal, 2013, 110, 731-738), ou la kuwanone C identifiée dans des feuilles de *Morus alba (*Characterization of Melanogenesis Inhibitory Constituents of Morus alba Leaves and Optimization of Extraction Conditions Using Response Surface MethodologyMolecules ; Jeong JY, et al; Molecules 2015 May 14;20(5):8730-41).

La wittiorumine F et la mulberrofurane T sont des adduits de type Diels-Alder (adduit d'une chalcone et d'un 2-arylbenzofurane prenylé). Le premier adduit a été isolé dans l'écorce de la tige de *Morus wittiorum (*Wittiorumins A - F, antioxidant diels-alder-type adducts from Morus wittiorum ; Tan YX et al; Planta Med. 2009 Feb;75(3):249-55) et le second adduit dans des cals de tissus de *Morus alba* ou dans l'écorce de racine de *Morus mongolica* (Five new diels-alder type adducts from the stem and root bark of *Morus mongolica ;* Kang J. et al ; Planta Med. 2006 Jan;72(1):52-9). La demande WO2013181296 décrit une composition comprenant des adduits de type Diels-Alder entre une chalcone et un groupement prénylphényle, et au moins un autre agent de gestion du poids pour traiter, prévenir ou gérer le gain de poids d'un mammifère.

Pour faire face à une demande croissante dans les domaines de la cosmétique et pharmaceutique, il est d'un grand intérêt d'identifier de nouveaux composés naturels capables d'inhiber de façon très efficace l'activité de la tyrosinase.

Pour répondre à ce besoin, les inventeurs ont maintenant développé un procédé permettant de préparer un extrait racinaire de plantes du genre *Morus* riche en polyphénols prenylés. Les inventeurs ont en effet montré que la culture de plantes du genre *Morus,* en particulier *Morus alba* et *Morus nigra,* dans des conditions particulières, permet d'obtenir un extrait racinaire desdites plantes riche en polyphénols prenylés avec la présence de wittiorumine F et de mulberrofurane T. Pour ces deux derniers composés, les inventeurs ont montré pour la première fois une affinité pour la tyrosinase. De façon étonnante, cette affinité est plus forte que celles d'inhibiteurs déjà connus comme la moracénine A, la moracénine B et la kuwanone C. Conformément aux résultats sur l'affinité, la wittiorumine F présente la meilleure activité inhibitrice, suivie de la kuwanone C, puis de la moracénine B et A. De manière inattendue, la moracénine A et B, la kuwanone C, la wittiorumine F et la mulberrofurane T présents dans l'extrait racinaire présentent également une affinité pour la collagénase, confirmée par la forte activité inhibitrice de la collagénase de l'extrait. De manière inattendue également, la moracénine A et B, la kuwanone C, la wittiorumine F et la mulberrofurane T présentes dans l'extrait racinaire présentent également une affinité pour la hyaluronidase, confirmée par la forte activité inhibitrice de la hyaluronidase de l'extrait, et notamment la forte activité de la moracénine A.

Un tel procédé permet aussi la réalisation d'extractions successives sans détruire, ni altérer la survie des plantes.

Enfin, les inventeurs ont montré que l'extrait racinaire selon l'invention en complément de ses bénéfices cosmétiques, comme le blanchiment ou un effet anti-âge comme par exemple un effet sur la résistance, la fermeté, la densité, l'élasticité ou encore sur l'aspect lisse de la peau (inhibition de la hyaluronidase et inhibition de la collagénase),

peut aussi être utilisé en tant que médicament pour favoriser la cicatrisation cutanée, en particulier dans le cas de la fermeture d'une plaie.

## DESCRIPTION DES FIGURES

Figures 1A à 1C : Chromatogrammes (UV à 265 nm) des extraits préparés à partir de racines de *M. alba non* stimulées (figure 1A) ou stimulées (figure 1B) ou à partir d'écorces de racines séchées de *M. alba* (figure 1C), selon l'exemple 1, dilués 5 fois avant injection. Les pics N°1, 2, 3, 4 et 5 correspondent respectivement à la moracénine B, la kuwanone C, la moracénine A, la wittiorumine F et le mulberrofurane T, selon l'exemple 1.

Figure 2 : Histogramme montrant le pourcentage d'activité de la tyrosinase en fonction du facteur de dilution d'un extrait de racines non stimulées de *M. alba,* selon l'exemple 2. La concentration inhibitrice médiane ($CI_{50}$) a été évaluée pour le facteur de dilution 150, ce qui correspond à un extrait préparé à partir de 0,13 mg de matière sèche dans 1 mL d'éthanol pur.

Figures 3A et 3B : Chromatogrammes (UV à 290 nm) d'un échantillon de référence (figure 3A) et du surnageant obtenu selon l'exemple 2.3 lors de l'évaluation de l'affinité d'un extrait de racines non stimulées de *M. alba* pour la tyrosinase, par la méthode dite de Target Binding® décrite dans la demande de brevet FR1670545 (figure 3B). Dans la figure 3A, les pics numérotés correspondent respectivement aux composés : moracénine B (1), kuwanone C (2), moracénine A (3), wittiorumine F (4) et mulberrofurane T (5).

Figures 4A et 4B : Chromatogrammes (UV à 290 nm) d'un échantillon de référence (figure 4A) et du surnageant obtenu selon l'exemple 3.2 lors de l'évaluation de l'affinité d'un extrait de racines non stimulées de *M. alba* pour la collagénase, par la méthode dite de Target Binding® (figure 4B). Sur la figure 4A, les pics numérotés correspondent respectivement aux composés : moracénine B (1), kuwanone C (2), moracénine A (3), wittiorumine F (4) et mulberrofurane T (5).

Figure 5 : Histogramme montrant les teneurs en moracénine B ainsi qu'en moracénine A, kuwanone C, wittiorumine F et mulberrofurane T, exprimées en équivalent moracénine B, dans l'extrait de racines stimulées de *M. alba* en fonction de la durée de la macération racinaire, selon l'exemple 5. Pour chacune des conditions de macération, les concentrations respectives (en mg/L) en moracénine B, kuwanone C (en équivalent moracénine B), moracénine A (en équivalent moracénine B), wittiorumine F (en équivalent moracénine B), mulberrofurane T (en équivalent moracénine B) sont indiquées de gauche à droite.

Figures 6A et 6B : Chromatogrammes (UV à 290 nm) d'un échantillon de référence (figure 6A) et du surnageant obtenu selon l'exemple 6.2 lors de l'évaluation de l'affinité d'un extrait de racines non stimulées de *M. alba* pour la hyaluronidase, par la méthode dite de Target Binding® décrite dans la demande de brevet FR1670545 (figure 6B). Sur les figures 6A et 6B, les pics numérotés correspondent respectivement aux composés : moracénine B (1), kuwanone C (2), moracénine A (3), wittiorumine F (4) et mulberrofurane T (5).

## DESCRIPTION DETAILLEE DE L'INVENTION

L'invention est définie par les revendications. La présente invention a pour objet un extrait racinaire de plantes du genre *Morus*, ladite plante étant choisie dans le groupe constitue par Morus alba et Morus nigra, caractérisé en ce :

- il comprend au moins 2 % en poids de moracénine B, de préférence au moins 2,3 %, au moins 3 %, au moins 4 %, au moins 5 %, au moins 6 % et plus préférentiellement au moins 7 %, exprimé par rapport au poids total de l'extrait sec,
- il comprend de la moracénine A, présente en une quantité d'au moins 0,05 % en poids d'équivalent moracénine B, de préférence au moins 0,1 %, au moins 0,5 %, au moins 1 %, au moins 1,5 % et plus préférentiellement au moins 2 %, exprimée par rapport au poids total de l'extrait sec,
- il comprend de la kuwanone C, présente en une quantité d'au moins 0,1 % en poids d'équivalent moracénine B, de préférence au moins 0,5 %, au moins 1 %, au moins 1,5 %, au moins 2 %, et plus préférentiellement au moins 2,5 %, exprimée par rapport au poids total de l'extrait sec, et
- il comprend au moins un des deux composés suivants :
    - de la wittiorumine F, présente en une quantité d'au moins 0,1 % en poids d'équivalent moracénine B, de préférence au moins 0,4 %, au moins 0,5 %, au moins 0,6 %, au moins 0,7 % et plus préférentiellement au moins 0,8 %, exprimée par rapport au poids total de l'extrait sec,
    - du mulberrofurane T, présent en une quantité d'au moins 0,1 % en poids d'équivalent moracénine B, de préférence au moins 0,2 %, au moins 0,3 %, au moins 0,4 % et plus préférentiellement au moins 0,5 %, exprimée par rapport au poids total de l'extrait sec.

Par « extrait racinaire de plantes du genre *Morus»* on entend un produit obtenu par l'extraction de racines de plantes du genre *Morus.* Ladite extraction peut être réalisée par tout moyen connu de l'homme du métier, et de préférence par l'un des moyens décrits dans la présente demande de brevet. De préférence on entend un produit obtenu par l'extraction de racines de plantes du genre *Morus* cultivées en conditions hors-sol, et particulièrement en aéroponie.

Par « dérivés prenylés de polyphénols » on entend l'ensemble des composés moracénine B, moracénine A, kuwanone C, wittiorumine F et mulberrofurane T.

Par « moracénine B » (aussi appelé kuwanone G) on entend un composé répondant à la formule générale (I) suivante : $C_{40}H_{36}O_{11}$

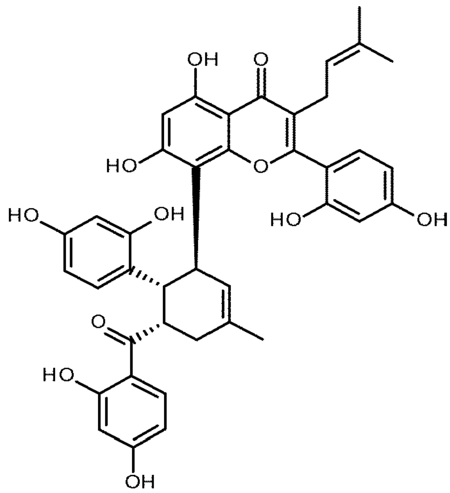

(I)

Par « moracénine A » (aussi appelé kuwanone H ou albaline G) on entend un composé répondant à la formule générale (II) suivante : $C_{45}H_{44}O_{11}$

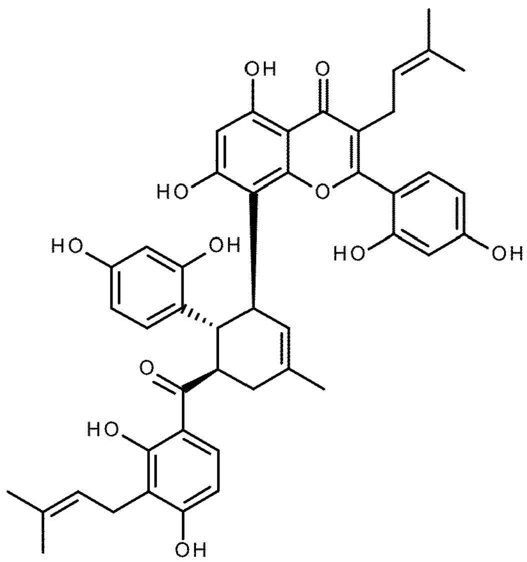

(II)

Par « kuwanone C » on entend un composé répondant à la formule générale (III) suivante : $C_{25}H_{26}O_6$

(III)

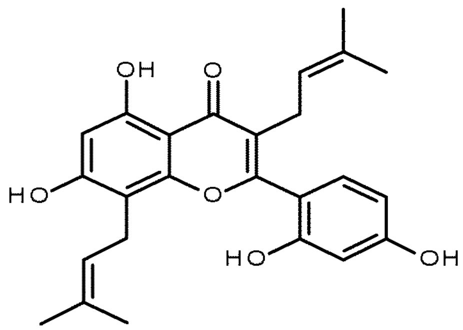

Par « wittiorumine F » on entend un composé répondant à la formule générale (IV) suivante : $C_{39}H_{36}O_9$. (IV)

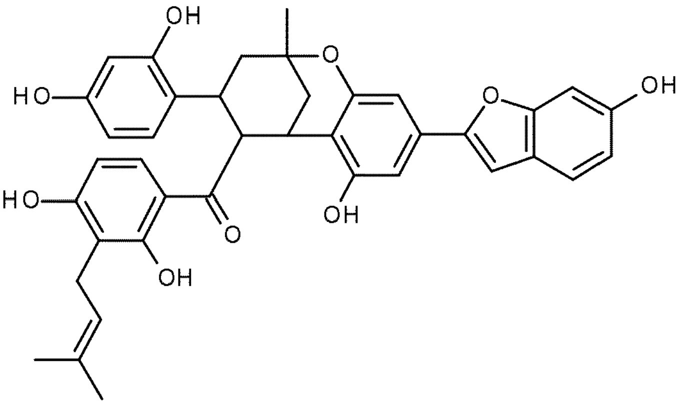

(IV)

Par « mulberrofurane T » on entend un composé répondant à la formule générale (V) suivante : $C_{44}H_{44}O_9$

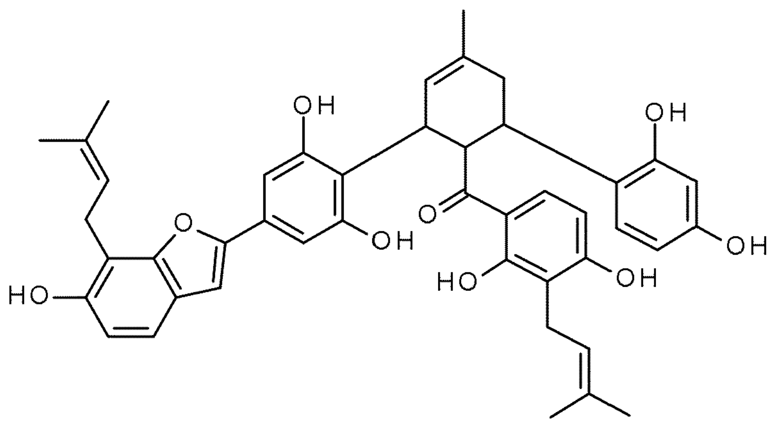

(V)

Par « extrait sec » on entend un extrait obtenu par la mise en oeuvre de tout type de procédé d'extraction, y compris un procédé selon l'invention, suivie d'une étape de dessication de l'extrait, la dessication étant mise en oeuvre selon toute méthode bien connue de l'homme du métier, notamment le traitement de l'extrait en atmosphère chaude et sèche.

Selon un aspect préféré, un « extrait sec » est donc un extrait obtenu par la mise en oeuvre du procédé selon l'invention suivie d'une étape de dessication de l'extrait.

La concentration dans un extrait racinaire d'un composé choisi parmi : la moracénine A, la kuwanone C, la wittiorumine F et le mulberrofurane T est déterminée par la mesure de l'aire du pic correspondant audit composé sur le

chromatogramme de l'analyse HPLC dudit extrait racinaire : l'aire dudit pic est rapportée à l'aire du pic correspondant à la moracénine B sur le chromatogramme de l'analyse HPLC d'une solution standard comprenant 100 mg/L de moracénine B ; un « équivalent moracénine B » est donc égal à :

$$100 \text{ x} \frac{(\text{aire du pic correspondant au composé de l'extrait})}{(\text{aire du pic correspondant à la moracénine B de la solution standard})} \; [mg/L]$$

Pour l'obtention d'un extrait racinaire, quand les racines sont jugées suffisamment développées, celles-ci sont mises en contact avec un solvant optionnellement par immersion ou de préférence par macération, et ensuite ledit solvant est récupéré et traité pour en extraire les composés d'intérêts qui ont été libérés par les racines desdites plantes. Ce type de procédé est adapté du procédé développé par la société Plant Advanced Technologies (PAT) sous le nom de « PAT Plantes à Traire® » et décrit dans la demande internationale WO 01/33942.

Un extrait racinaire de plantes du genre *Morus* selon l'invention est un extrait racinaire d'une plante du genre *Morus* choisie parmi *Morus alba* et *Morus nigra.* De façon plus préférentielle il s'agit d'un extrait racinaire de *Morus alba.* Selon un aspect particulier, un extrait racinaire de plantes du genre *Morus* selon l'invention est un extrait racinaire d'une plante du genre *Morus* choisie parmi *Morus alba* et *Morus nigra* cultivée en conditions hors-sol, et particulièrement en aéroponie.

Dans un deuxième aspect, l'invention a pour objet un procédé de préparation d'un extrait racinaire de plantes du genre *Morus* selon l'invention, comprenant

a) une étape de culture de plantes du genre *Morus* en conditions hors-sol, de préférence en aéroponie,
b) optionnellement une étape de stimulation des racines des plantes,
c) une étape d'extraction solide/liquide des racines optionnellement stimulées lors de l'étape b), et
d) la récupération de l'extrait obtenu lors de l'étape c).

Dans le cadre de l'invention, on entend par « culture des plantes en conditions hors sol », tout mode de culture dans lequel les racines de la plante ne sont pas dans de la terre. Plus précisément, la culture hors sol est une culture dans laquelle les racines des plantes reposent dans un milieu reconstitué, détaché du sol. Ce milieu de culture est irrigué de façon régulière par des solutions nutritives appropriées à la plante cultivée.

Il existe différentes techniques de culture hors sol tels que les systèmes sans substrat qui nécessitent une solution nutritive enrichie en oxygène, et les systèmes avec substrat. Parmi les systèmes sans substrat, on peut citer l'aquiculture pour laquelle la solution nutritive est non circulante et est contenue dans un bac de culture, la technique de culture sur film nutritif (en anglais Nutrient Film Technique ou N.F.T.) pour laquelle la solution nutritive s'enrichit en oxygène dissous au cours de son déplacement par échange avec l'air, et l'aéroponie. Parmi les systèmes avec substrat, on retrouve la subirrigation dans laquelle la solution nutritive pénètre dans le substrat au niveau de sa partie inférieure et la percolation dans laquelle la solution nutritive est distribuée par irrigation discontinue à la surface supérieure du système puis percole vers le bas du substrat. Le substrat, minéral ou organique, est neutre et inerte comme du sable, de l'argile ou de la laine de roche par exemple. Ce substrat peut être également d'origine industrielle.

On entend par « culture des plantes en aéroponie » un mode de culture hors sol pour lequel les racines des plantes ne sont en contact ni avec un milieu solide, ni même avec un milieu liquide. Selon un mode de réalisation particulier, les plantes sont alimentées par un brouillard nutritif obtenu par brumisation, via un brumisateur, de la solution nutritive dans un milieu fermé.

Typiquement, dans un procédé selon l'invention, on peut disposer des plantes sur des plateaux avec la partie aérienne de la plante au-dessus du plateau et la partie racinaire en-dessous, on dispose les plateaux sur des tables formant une zone de rétention pour collecter l'excédent d'un liquide diffusé vers les plantes, et on transfère les plateaux sur les tables à différents postes.

Lors de l'étape a), les plantes cultivées en aéroponie sont alimentées par pulvérisation racinaire d'une solution nutritive de sels minéraux essentiels (Azote - N, Phosphore - P, Potassium - K), afin d'obtenir un développement racinaire maximum et une concentration maximum en composés d'intérêt, sans altérer la survie de la plante. L'homme du métier, à l'aide de ses connaissances générales, sait comment adapter les proportions et concentrations des différents sels minéraux pour optimiser le développement racinaire et la concentration des composés d'intérêt. Les concentrations en sels minéraux des solutions nutritives sont dans ce cas comprises dans une gamme d'électroconductivité s'étendant avantageusement entre 0,8 à 1,6 mS/cm, de préférence entre 1 à 1,2 mS/cm, afin de permettre une croissance maximale de la plante avec un meilleur rendement en biomasse racinaire ainsi que de favoriser une plus grande teneur en polyphénols prenylés dans les extraits.

Dans l'étape c) d'un procédé selon l'invention, on entend par « extraction solide/liquide » toute technique d'extraction par solvant qui consiste à extraire une espèce chimique se trouvant dans un solide et étant soluble dans

ledit solvant. Parmi ces techniques d'extraction, on peut citer la macération, l'immersion, l'exsudation, l'infusion, la décoction, l'extraction par extracteurs de Soxhlet et de Kumagawa, l'extraction assistée par micro-onde, l'extraction assistée par ultrason, l'extraction par voie enzymatique, l'extraction par fluide supercritique (CO2 + DiPropylèneGlycol). L'extraction conduit à la récupération des métabolites contenus dans l'une ou l'autre partie des plantes, et notamment les racines, au moyen d'un liquide de lessivage, ou solvant, mis en contact avec les racines coupées dans un solvant particulier et pendant une durée appropriée.

Selon un aspect particulier, dans un procédé selon l'invention, l'étape c) d'extraction solide/liquide des racines optionnellement stimulées lors de l'étape b), est réalisée au moyen d'une macération.

Selon un aspect particulier, dans un procédé selon l'invention, l'étape c) d'extraction solide/liquide des racines optionnellement stimulées lors de l'étape b), comprend la coupe des racines.

Typiquement, les plantes sont disposées sur un plateau avec la partie aérienne de la plante au-dessus du plateau et la partie racinaire au-dessous. Une étape de coupe dans laquelle la partie racinaire des plantes est partiellement coupée permet de récolter les racines coupées. Il est possible d'extraire les substances des racines coupées.

Selon un autre aspect préféré, la macération racinaire est réalisée sur des racines fraîchement coupées, c'est-à-dire coupées depuis moins de 24 heures, et de préférence le plus tôt possible après coupage, idéalement juste après coupage. En particulier, la macération racinaire peut être effectuée grâce à une étape de macération des racines fraîchement coupées dans un solvant approprié, à un pH approprié, et pendant une durée appropriée.

De préférence, la macération racinaire est réalisée sur des racines coupées et éventuellement séchées. Selon un autre aspect plus particulier, la macération racinaire est réalisée sur des racines coupées, éventuellement séchées, puis broyées. Le séchage des racines peut être réalisé par la mise en oeuvre de tout procédé de séchage adapté, connu de l'homme du métier, et notamment en plaçant les racines à une température comprise entre 30°C et 50°C pendant 4 heures à 72 heures, de préférence dans un environnement sec. Le séchage des racines peut notamment être réalisé dans une étuve ventilée. Le broyage des racines peut être réalisé par la mise en oeuvre de tout procédé de broyage adapté, connu de l'homme du métier, et notamment en plaçant les racines dans un broyeur à billes, un broyeur à couteaux ou un broyeur à cylindres. Par exemple les racines séchées peuvent être broyées jusqu'à l'obtention d'une poudre.

Plus particulièrement, l'étape c) d'un procédé selon l'invention comprend la mise en présence des racines avec un solvant choisi parmi : les alcools, les glycols et les solvants eutectiques. Ledit alcool est choisi de préférence parmi l'éthanol et le méthanol, utilisés purs ou sous la forme d'une solution aqueuse d'alcool, celle-ci comprenant de 10% à 99,9% d'alcool, plus particulièrement entre 40% et 90%, et encore plus particulièrement entre 50% et 85%. Ledit glycol est un diol dans lequel les deux groupes hydroxyles sont portés par des carbones différents, et est choisi parmi : le dipropylène glycol, le propane-1,3-diol, le propane-1,2-diol et le butylène glycol, et est utilisé pur ou sous la forme d'une solution aqueuse de glycol, celle-ci comprenant de 10% à 99,9% de glycol, plus particulièrement entre 40% et 90%, et encore plus particulièrement entre 50% et 85%. Ledit solvant eutectique est constitué de deux composants ou plus, qui peuvent être solides ou liquides et qui, dans une composition particulière, présentent une forte diminution de leur température de fusion, rendant ainsi le mélange liquide. Les solvants eutectiques naturels sont principalement constitués d'acides aminés, d'acides organiques, de sucres et de dérivés de la choline. L'eau peut faire partie du solvant, mais est dans ce cas fortement retenu dans le liquide et ne peut être évaporée. Les combinaisons particulières des composants constituant ledit solvant eutectique sont choisies parmi notamment, et de manière non exhaustive, le chlorure de choline - glucose (ratio 1:1), le chlorure de choline - acide citrique (ratio 1:1), le chlorure de choline - acide citrique (ratio 2:1), le chlorure de choline - saccharose (ratio 4:1), le chlorure de choline - saccharose (ratio 1:1), le chlorure de choline - acide tartrique (ratio 2:1), le chlorure de choline - xylose (ratio 2:1), le chlorure de choline - xylose (ratio 3:1), l'acide citrique - saccharose (ratio 1:1), l'acide citrique - glucose (ratio 1:1), le glucose - acide tartrique (ratio 1:1), le chlorure de choline - urée (ratio 1:2), le chlorure de choline - xylitol - eau (2 : 1 : 3) et l'acide lactique - glucose - eau (5 : 1 : 3).

Par « butylène glycol » on entend le butane-1,2-diol, le butane-1,3-diol, le butane-2,3-diol et le butane-1,4-diol.

Ces solvants, et plus particulièrement l'éthanol, le butylène glycol, le propane-1,3-diol et le propane-1,2-diol permettent de favoriser l'extraction d'une forte teneur en polyphénols prenylés.

Dans le cas d'un mélange d'au moins deux extraits racinaires, les solvants présents dans lesdits au moins deux extraits peuvent être identiques ou différents.

Selon un aspect particulier de l'invention, le solvant est caractérisé par un pH acide à faiblement acide, notamment lorsqu'il est utilisé lors de l'étape d'extraction solide/liquide. Selon un aspect particulier, ledit alcool ou ledit glycol utilisé lors d'une étape d'extraction solide/liquide est caractérisé par un pH compris entre 3,5 et 6,5, de préférence un pH égal ou supérieur à 4 et inférieur ou égal à 6,5, plus préférentiellement un pH égal ou supérieur à 4 et inférieur ou égal à 6, encore plus préférentiellement un pH compris entre 4 et 4,5. L'utilisation d'un solvant à pH acide à faiblement acide, c'est-à-dire supérieur ou égal à 3,5 et inférieur ou égal à 6,5, favorise la solubilisation des composés d'intérêt dans ledit solvant.

Selon un autre aspect particulier, un extrait végétal après extraction selon l'invention est caractérisé par un pH compris entre 5 et 7 et de préférence entre 5,5 et 6,5. Un pH faiblement acide à neutre limite la dégradation chimique

ou enzymatique des polyphénols prenylés après l'extraction.

Les acides utilisés pour l'ajustement du pH du solvant ou de l'extrait sont de préférence l'acide lactique, l'acide phosphorique, l'acide citrique ou l'acide chlorhydrique.

Plus particulièrement, ledit glycol est choisi dans le groupe suivant : butylène glycol, le propane-1,3-diol et le propane-1, 2-diol.

Le propane-1,3-diol, ou triméthylène glycol, peut être préparé par synthèse chimique selon des techniques connues de l'homme du métier et décrites dans la littérature, il est aussi disponible commercialement. Ledit propane-1,3-diol peut également être produit par la mise en oeuvre d'un procédé de fermentation en conditions adaptées d'un organisme vivant, notamment une souche génétiquement modifiée *d'Escherichia coli.* Le propane-1,3-diol ainsi obtenu est désigné par le terme de « propane-1,3-diol biosourcé », un tel produit est produit puis purifié comme décrit notamment dans la demande internationale WO 2004/101479 et commercialisé sous la marque Zéméa®. Ledit propane-1,3-diol peut également avoir une certification Bio, par exemple de type COSMOS.

De façon encore plus particulière, dans un procédé selon l'invention, l'étape c) d'extraction solide/liquide comprend la mise en présence des racines avec du propane-1,2-diol ou avec du propane-1,3-diol, et notamment du propane-1,3-diol biosourcé et/ou de certification Bio.

Les durées de macération racinaire sont choisies de manière à favoriser l'extraction d'une forte quantité en composés d'intérêt.

Selon un aspect particulier, l'étape c) d'un procédé selon l'invention comprend la mise en présence des racines avec un solvant pendant une durée comprise entre 5 minutes et 3 heures pour la macération de racines séchées et pendant une durée comprise entre 15 minutes et 96 heures, notamment entre 24 heures et 72 heures, plus particulièrement entre 24 heures et 48 heures pour la macération de racines fraîches.

Pour la macération racinaire de racines fraîches, le ratio de la quantité de racines fraîches sur la quantité de solvant varie entre 0,2 kg de racines / L de solvant à 1 kg de racines / L de solvant.

Selon un autre aspect particulier, un procédé selon l'invention comprend une étape de séchage des racines fraîches coupées, préalablement à l'étape de macération desdites racines, la macération étant réalisée par la mise en présence des racines séchées avec un solvant. Pour la macération racinaire, le ratio de la quantité de racines sèches sur la quantité de solvant varie entre 0,01 kg de racines / L de solvant à 0,1 kg de racines / L de solvant.

De préférence l'étape de macération des racines par la mise en présence des racines avec un solvant, est réalisée sous agitation.

Selon un aspect particulier, les plantes, après l'étape de coupe des racines, sont remises en culture pendant 1 à 8 semaines en aéroponie selon les étapes a) et optionnellement soumises pendant 1 jour à 8 semaines à une étape de stimulation b) afin de recommencer leur développement racinaire et favoriser la production par les racines de métabolites secondaires.

Selon un aspect préféré, un procédé selon l'invention est un procédé de préparation d'un extrait racinaire de plantes de l'espèce *Morus alba* ou de l'espèce *Morus nigra,* plus préférentiellement de l'espèce *Morus alba* ou de l'espèce *Morus nigra,* cultivées en conditions hors-sol, et particulièrement en aéroponie.

Selon un mode de réalisation préféré, l'invention a également pour objet un procédé de préparation d'un extrait racinaire de plantes du genre *Morus* selon l'invention comprenant :

a) une étape de culture de plantes du genre *Morus* en conditions hors-sol, et particulièrement en aéroponie,
b) une étape de stimulation des racines des plantes,
c) une étape d'extraction solide/liquide des racines stimulées lors de l'étape b), et
d) la récupération de l'extrait obtenu lors de l'étape c).

Selon un autre aspect particulier, dans un procédé selon l'invention, l'étape b) de stimulation des racines des plantes comprend :

- une étape d'élicitation dans laquelle les racines sont mises en présence d'une solution comprenant au moins un agent choisi parmi : un sel, un tensioactif, un solvant, un éliciteur d'origine fongique, végétale ou bactérienne, un dérivé de l'acide jasmonique, en particulier le méthyl jasmonate, l'acide salicylique, un générateur d'éthylène, la coronalone, une chitine, des chitosans et/ou leur mélange, et/ou
- une étape de mise en présence des racines avec une solution carencée en azote, c'est-à-dire une solution comprenant une proportion d'azote inférieure à la proportion d'azote habituellement considérée comme optimale pour le développement de la plante, avantageusement moins de 15% d'azote, et avantageusement ne comprenant pas d'azote, lesdites étapes étant séquentielles ou simultanées.

Les racines peuvent également être stimulées par la mise en présence des plantes avec une solution nutritive carencée en azote. La mise en présence des plantes avec une solution nutritive carencée en azote provoque un « stress

azoté » responsable de la stimulation. Selon un aspect particulier, une solution carencée en azote selon la présente invention est une solution comprenant moins de 15% d'azote, de préférence moins de 10% d'azote, avantageusement moins de 8%, plus avantageusement moins de 6%, moins de 5%, moins de 4%, moins de 3%, moins de 2%, moins de 1% d'azote et encore plus avantageusement 0% d'azote.

L'étape b) de stimulation permet d'augmenter de manière significative la teneur en métabolites secondaires dans les racines et favoriser ainsi le flux des métabolites sortant des racines vers le solvant choisi pour l'extraction et ce, sans perte totale de la viabilité de la plante afin qu'elle puisse être remise en culture puis réutilisée. En d'autres termes, l'étape de stimulation de la plante permet de favoriser la production et la sécrétion des composés d'intérêt.

Avantageusement, l'étape b) de stimulation des racines est réalisée par pulvérisation ou macération de la plante avec une solution d'éliciteurs choisie parmi : l'acide salicylique, la coronalone et les chitosans ou par l'alimentation de la plante avec une solution nutritive N/P/K carencée en azote vaporisée sur les racines.

Selon un mode particulier de réalisation de l'invention, l'étape b) est mise en oeuvre par pulvérisation ou macération de la plante avec une solution choisie parmi :

- une solution de corolanone à une concentration comprise entre 0,1 et 200 μM, avantageusement entre 1 et 100 μM,
- une solution d'acide salicylique à une concentration comprise entre 1 et 500 μM, avantageusement entre 10 et 50 μM,
- une solution de chitosans à une concentration de 0,002 à 1 g/L, avantageusement de 0,05 à 0,1 g/L, et
- une solution nutritive N/P/K comprenant moins de 6% d'azote, ladite solution étant de préférence vaporisée sur les racines.

En outre, l'étape b) de stimulation des racines avec une solution d'éliciteurs est avantageusement effectuée pendant une durée comprise entre 1 jour et 21 jours, de préférence entre 1 et 7 jours.

Selon un aspect particulier, l'étape b) de stimulation des racines par l'alimentation de la plante avec une solution nutritive N/P/K carencée en azote vaporisée sur les racines est avantageusement effectuée pendant une durée comprise entre 7 jours et 8 semaines, de préférence 3 semaines.

Lors de l'étape b), les concentrations en sels minéraux des solutions nutritives sont comprises dans une gamme d'électroconductivité s'étendant avantageusement entre 0,6 à 0,9 mS/cm, de préférence entre 0,6 à 0,8 mS/cm, afin de favoriser une plus grande teneur en polyphénols prenylés des extraits.

Selon un aspect particulier d'un procédé selon l'invention, l'étape b) est réalisée après l'étape a). Selon un autre aspect particulier d'un procédé selon l'invention, l'étape b) et l'étape a) sont effectuées de manière simultanée, la solution de stimulation est alors incorporée à la solution nutritive ou administrée par tout autre mode d'administration connu de l'homme du métier.

Selon un perfectionnement, l'étape de « coupe » des racines est précédée d'une étape de lavage dans laquelle le liquide diffusé vers les racines est de l'eau claire. On limite ainsi l'apport des éléments contenus dans la solution nutritive ou dans l'éventuelle solution de stimulation lors de l'étape d'extraction solide/liquide.

Avantageusement, l'invention a pour objet un procédé de préparation d'un extrait racinaire de plantes du genre *Morus* comprenant les étapes suivantes :

a) une étape de culture de plantes du genre *Morus* en conditions hors-sol, et particulièrement en aéroponie,
b) une étape de stimulation des racines des plantes,
c) une étape d'extraction solide/liquide des racines stimulées lors de l'étape b), comprenant la coupe, le séchage et le broyage desdites racines, suivis de la macération des racines broyées dans un solvant, ledit solvant étant choisi parmi : les alcools, les glycols et les solvants eutectiques, et étant utilisé pur ou sous la forme d'une solution aqueuse d'alcool, de glycol ou de solvant eutectique, et
d) la récupération des extraits obtenus lors de l'étape c).

Selon un aspect particulier, dans un procédé selon l'invention le pH du solvant est compris entre 3,5 et 6,5, de préférence entre 3,5 et 6, de préférence entre 3,5 et 5, plus préférentiellement entre 4 et 4,5.

Un procédé selon l'invention comprend avantageusement une étape supplémentaire de réajustement de la teneur en solvant de l'extrait, pour obtenir une teneur avantageuse d'au moins 50%, et/ou un ajustement du pH de l'extrait, pour obtenir un pH avantageusement compris entre 3 et 7, de préférence compris entre 4 et 6,5, en vue de la conservation de l'extrait.

Un procédé selon l'invention comprend avantageusement au moins une étape supplémentaire de purification et/ou de traitement de l'extrait racinaire, une telle étape est notamment choisie parmi :

- au moins une filtration, en particulier une nanofiltration et/ou une filtration stérilisante et/ou une filtration clarifiante,
- une extraction liquide/solide,
- une purification,

- une concentration et
  - une décoloration de l'extrait racinaire,

de telles méthodes de purification et/ou de traitement sont bien connues de l'homme du métier. Cette étape permet d'obtenir l'extrait final de *Morus,* de préférence de *Morus alba* ou *Morus nigra,* riche en polyphénols prenylés, notamment en moracénine B, en moracénine A, en kuwanone C, en wittiorumine F et en mulberrofurane T.

La figure 5 montre les différentes concentrations en moracénine B, en kuwanone C, en moracénine A, en wittiorumine F et en mulberrofurane T dans un extrait racinaire de *M. alba* selon les durées de macération selon l'invention.

La présente invention a également pour objet un extrait racinaire susceptible d'être obtenu par un procédé selon l'invention.

La présente invention a pour troisième objet une composition cosmétique comprenant, en tant qu'agent actif, en une quantité comprise entre 0,01 et 10 % en poids par rapport au poids total de la composition, au moins un extrait racinaire selon l'invention ou un extrait racinaire obtenu par un procédé selon l'invention, et au moins un excipient cosmétiquement acceptable. Avantageusement, ledit extrait racinaire est un extrait de plantes du genre *Morus,* de préférence *Morus alba* ou *Morus nigra.* De façon plus préférentielle il s'agit d'un extrait racinaire de *Morus alba.* Selon un aspect particulier l'un extrait racinaire de plantes du genre *Morus* est un extrait racinaire d'une plante du genre *Morus* choisie parmi *Morus alba* ou *Morus nigra* cultivées en conditions hors-sol, et particulièrement en aéroponie.

Les modes d'administration, les posologies et les formes galéniques optimales d'une composition cosmétique selon l'invention peuvent être déterminés selon les critères généralement pris en compte dans l'établissement d'un traitement cosmétique adapté à un sujet comme par exemple le type de peau.

La composition cosmétique selon l'invention est avantageusement destinée à une application topique. Elle peut notamment se présenter sous la forme d'une crème, d'un lait, d'une lotion, d'un gel, d'un sérum, d'un spray, d'une mousse, d'une solution, d'une pommade, d'une émulsion, d'un patch ou d'un masque. Une composition cosmétique selon l'invention comprend au moins un excipient cosmétiquement acceptable choisi en fonction du type d'administration souhaitée. Une composition cosmétique selon la présente invention peut en outre comprendre au moins un adjuvant connu de l'homme du métier, choisi parmi les épaississants, les conservateurs, les parfums, les colorants, des filtres chimiques ou minéraux, les agents hydratants, les eaux thermales, etc.

Un excipient cosmétiquement acceptable peut être choisi parmi les polymères, les composés siliconés, les agents tensioactifs, les agents de rhéologie, les agents humectants, les agents de pénétration, les composants huileux, les cires, les émulsifiants, les agents filmogènes, les parfums, les électrolytes, les ajusteurs de pH, les agents antioxydants, les conservateurs, les colorants, les nacres, les pigments et leurs mélanges.

Une composition cosmétique selon l'invention peut en outre comprendre au moins un autre agent cosmétiquement actif, tels qu'un autre agent anti-âge, un agent hydratant, un agent ayant une activité calmante, apaisante ou relaxante, un agent stimulant la microcirculation cutanée, un agent séborégulateur pour le soin des peaux grasses, un agent nettoyant ou purifiant, un agent anti-radicalaire, un agent anti-inflammatoire, un filtre solaire chimique ou minéral, etc.

Avantageusement, une composition cosmétique selon l'invention comprend au moins un extrait racinaire de *Morus* selon l'invention, et en particulier un extrait racinaire de *Morus alba* ou de *Morus nigra* selon l'invention, en une quantité comprise entre 0,01 et 10%, en particulier entre 0,05 et 5%, plus particulièrement entre 0,1 et 2%, en poids par rapport au poids total de la composition.

Une composition cosmétique selon l'invention peut notamment être destinée à avoir un effet blanchiment de la peau en inhibant notamment l'activité de la tyrosinase.

Une composition cosmétique selon l'invention peut aussi être destinée à avoir un effet anti-âge, en inhibant notamment l'activité de la hyaluronidase et/ou en inhibant l'activité de la collagénase.

La présente invention a pour quatrième objet un extrait racinaire selon l'invention, un extrait racinaire obtenu par un procédé selon l'invention, ou une composition cosmétique selon l'invention, pour prévenir ou retarder l'apparition des effets du vieillissement de la peau en stimulant la fonction barrière de l'épiderme et/ou en inhibant l'activité de la hyaluronidase, et/ou en inhibant l'activité de la collagénase, et/ou avoir un effet blanchiment de la peau.

La présente invention a également pour objet une utilisation d'un extrait selon l'invention, un extrait obtenu par un procédé selon l'invention pour la préparation d'une composition cosmétique visant à prévenir ou retarder l'apparition des effets du vieillissement de la peau et/ou visant un effet blanchiment de la peau.

La présente invention a également pour objet une méthode de soin cosmétique de la peau visant à prévenir ou retarder l'apparition des effets du vieillissement de la peau, en stimulant la fonction barrière de l'épiderme, et/ou en inhibant l'activité de la hyaluronidase et/ou en inhibant l'activité de la collagénase, et/ou visant un effet blanchiment de la peau, ladite méthode étant caractérisée en ce qu'elle comprend l'application, sur au moins une partie de la peau du corps ou du visage, d'une composition cosmétique selon l'invention.

La présente invention a pour cinquième objet une composition pharmaceutique comprenant, en tant qu'agent

actif, en une quantité comprise entre 0,01 et 10 % en poids par rapport au poids total de la composition, au moins un extrait selon l'invention ou un extrait obtenu par un procédé selon l'invention, et au moins un excipient pharmaceutiquement acceptable.

Dans la présente invention, on désigne par « pharmaceutiquement acceptable » tout ingrédient qui est utile dans la préparation d'une composition pharmaceutique, qui est généralement sûr, non toxique et ni biologiquement ni autrement non souhaitable et qui est acceptable pour une utilisation vétérinaire ou chez l'Homme.

On désigne par « sels pharmaceutiquement acceptables » d'un composé, des sels qui sont pharmaceutiquement acceptables, comme défini ci-dessus, et qui possèdent l'activité pharmacologique souhaitée du composé parent. De tels sels comprennent :

(1) les hydrates et les solvates,

(2) les sels d'addition d'acide pharmaceutiquement acceptables formés avec des acides inorganiques pharmaceutiquement acceptables tels que l'acide chlorhydrique, l'acide bromhydrique, l'acide sulfurique, l'acide nitrique, l'acide phosphorique et similaires ; ou formés avec des acides organiques pharmaceutiquement acceptables tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires, ou

(3) les sels d'addition de base pharmaceutiquement acceptables formés lorsqu'un proton acide présent dans le composé parent est soit remplacé par un ion métallique, par exemple un ion de métal alcalin, un ion de métal alcalino-terreux ou un ion d'aluminium ; soit coordonné avec une base organique ou inorganique pharmaceutiquement acceptable. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium.

Les modes d'administration, les posologies et les formes galéniques optimales d'une composition pharmaceutique selon l'invention peuvent être déterminés selon les critères généralement pris en compte dans l'établissement d'un traitement pharmaceutique adapté à un sujet comme par exemple l'âge ou le poids corporel du patient, la gravité de son état général, la tolérance au traitement, les effets secondaires constatés, le type de peau. En fonction du type d'administration souhaitée, la composition pharmaceutique selon l'invention peut en outre comprendre au moins un excipient pharmaceutiquement acceptable. La composition pharmaceutique selon la présente invention peut en outre comprendre au moins un adjuvant pharmaceutiquement connu de l'homme du métier, choisi parmi les épaississants, les conservateurs, les parfums, les colorants, des filtres chimiques ou minéraux, les agents hydratants, les eaux thermales, etc.

Avantageusement, ladite composition pharmaceutique comprend au moins un extrait selon l'invention en une quantité comprise entre 0,01 et 10%, en particulier entre 0,05 et 5%, plus particulièrement entre 0,1 et 2%, en poids par rapport au poids total de la composition.

Une composition pharmaceutique est particulièrement adaptée pour une administration par voie orale, nasale, transdermique, parentérale, topique, rectale et mucosale. Elle peut se présenter sous forme sèche, telle que par exemple : capsule molle, gélule, comprimé, lyophilisat, poudre, granule, ou patch, ou sous forme liquide, telle que : solution, suspension, spray, crème ou gel.

L'excipient pharmaceutiquement acceptable est connu de l'Homme du Métier et est choisi selon le mode d'administration de la composition pharmaceutique. A titre d'exemple, l'excipient pharmaceutiquement acceptable peut être choisi dans le groupe constitué par les agents diluants, liants, désintégrants, les colorants, les lubrifiants, les agents solubilisants, les agents promoteurs d'absorption, les filmogènes, les agents gélifiants, et leurs mélanges.

La composition pharmaceutique selon l'invention peut en outre comprendre au moins un composé choisi dans le groupe constitué par les émollients, les actifs hydratants, les activateurs de la synthèse de kératine, les kératorégulateurs, les kératolytiques, les agents restructurant de la barrière cutanée (activateurs de la synthèse des lipides cutanés, agonistes PPARs ou Peroxysome Proliferator Activated Receptor), les activateurs de la différenciation des kératinocytes (rétinoïdes, calcidone®, le calcium), les antibiotiques, les agents anti-bactériens, les composés antifongiques, les agents anti-viraux, les sébo-régulateurs, les immunomodulateurs, tels que le tacrolimus, le pimécrolimus, les oxazolines, les conservateurs, les agents anti-irritants, les agents apaisants, des filtres et écrans solaires, les agents anti-oxydants, les facteurs de croissance, les agents cicatrisants ou les molécules eutrophiques, les médicaments et les agents anti-inflammatoires, les médicaments et les agents anti-Alzheimer.

La présente invention a pour septième objet un extrait selon l'invention ou une composition pharmaceutique

selon l'invention, pour son utilisation en tant que médicament.

Plus particulièrement, la présente invention a également pour objet un extrait selon l'invention, un extrait obtenu par un procédé selon l'invention, ou une composition pharmaceutique selon l'invention; pour son utilisation en tant que médicament pour favoriser la cicatrisation cutanée, en particulier dans le cas de la fermeture d'une plaie. Ledit extrait sera avantageusement associé à un excipient pharmaceutiquement acceptable et adapté pour une application cutanée, et ladite composition pharmaceutique contiendra avantageusement un excipient pharmaceutiquement acceptable et adapté pour une application cutanée.

Plus particulièrement, la présente invention a également pour objet un extrait selon l'invention, un extrait obtenu par un procédé selon l'invention, ou une composition pharmaceutique selon l'invention, pour son utilisation en tant que médicament pour stimuler et/ou améliorer la fermeté, la densité, la résistance, l'aspect lisse et/ou l'élasticité de la peau. Ledit extrait sera avantageusement associé à un excipient pharmaceutiquement acceptable et adapté pour une application cutanée, et ladite composition pharmaceutique contiendra avantageusement un excipient pharmaceutiquement acceptable et adapté pour une application cutanée.

La stimulation et/ou l'amélioration de la fermeté, de la densité, de la résistance, de l'aspect lisse et/ou de l'élasticité de la peau sont notamment dues à l'inhibition de l'activité de la hyaluronidase et/ou l'inhibition de l'activité de la collagénase.

L'invention concerne aussi une méthode de soin thérapeutique de la peau pour stimuler et/ou améliorer la fermeté, la densité, la résistance, l'aspect lisseet/ou l'élasticité de la peau et/ou pour favoriser la cicatrisation cutanée, en particulier dans le cas de la fermeture d'une plaie, chez un sujet en ayant besoin, comprenant l'administration au sujet d'une quantité thérapeutiquement efficace d'une composition pharmaceutique selon l'invention.

L'invention concerne aussi une méthode de soin cosmétique de la peau pour favoriser le blanchiment de la peau et/ou pour prévenir ou retarder l'apparition des effets du vieillissement (effet anti-âge), comprenant l'administration à un sujet d'une quantité cosmétiquement efficace d'une composition cosmétique selon l'invention.

Les exemples 1 à 7 qui suivent et les figures 1 à 6 visent à illustrer la présente invention, sans toutefois en limiter la portée.

## EXEMPLES

Les extraits racinaires de *Morus alba* sont préparés à partir de plantes dont les graines ont été achetées auprès d'un fournisseur français (Les semences du Puy) de 2012 à 2015. Les extraits racinaires de *Morus nigra* sont préparés à partir de plantes dont les graines ont été achetées auprès d'un fournisseur français (Naudet Pépinières) en 2016. Depuis 2016, les plantes de *Morus alba et de Morus nigra* sont conservées et multipliées en serre par la demanderesse.

## EXEMPLE 1 : PROFIL PHYTOCHIMIQUE D'UN EXTRAIT RACINAIRE DE MORUS ALBA ET DE MORUS NIGRA AVANT ET APRES STIMULATION

Des plants de *Morus alba* sont cultivées en aéroponie pendant 8 semaines avec un milieu de culture 15/10/30 (N/P/K) et à une électroconductivité comprise entre 1,0 et 1,2 mS/cm, puis avec une solution nutritive définie, de composition N/P/K correspondant à 0/15/40 et à une électroconductivité comprise entre 0,6 et 0,8 mS/cm pendant 3 semaines.

Des racines ont été récoltées avant le changement de milieu et elles correspondent à des racines non stimulées.

Des racines coupées et récoltées avant et après stimulation par carence azotée sont séchées pendant 48 heures à 40°C dans une étuve ventilée et broyées à l'aide d'un broyeur à billes (WWR star-beater) pendant 4 minutes à la vitesse de 15 battements par seconde. 20 mg de racines broyées à l'état de poudre sont mises à macérées dans 1 mL l'éthanol pur sous agitation (à l'aide d'un vortex) à température ambiante pendant 30 minutes. Les échantillons sont centrifugés à 21000 g pendant 10 minutes afin de séparer la matière solide. Les surnageants sont récupérés et dilués 5 fois dans de l'éthanol pur pour être injectés.

L'appareil utilisé pour l'étape d'analyse est une UPLC Shimadzu Nexera X2 (les pompes LC-30AD, passeur d'échantillon SIL-30AC, four CTO-20A, détecteurs à barrette de diodes SPD-M20A ; Kyoto, Japon) fonctionnant en phase inverse avec une colonne Kinetex EVO C18 (00F-4725-AN, Phenomenex, Torrance, CA, USA) de dimensions 150 mm $\times$ 2.1 mm, 2.6 $\mu$m. La phase mobile est constituée d'un solvant A (Eau ultrapure Mili-Q, Merck Millipore +0.1% d'acide formique, Carlo Erba, Val-de-Reuil, France) et d'un solvant B (Acetonitrile, Sigma-Aldrich Chemie GmbH, Steinheim, Allemagne) dont le gradient a été programmé de la façon suivante : phase B (%) 5-72,5% (0-22 min) ; 72,5-90% (22-22,1 min) ; 90% (22,1-23,9 min), 90-5% (23,9-24 min), 5% (24-26 min). Le débit d'analyse est de 0,5 mL/min avec une température de four de 40°C. En sortie de colonne, un détecteur à barrettes de diodes enregistre les spectres UV entre 220 et 370 nm. L'appareil est couplé à un spectromètre de masse (Shimadzu LCMS-2020) fonctionnant avec une ionisation par électrospray (4,5 kV) en mode négatif dans une gamme de m/z entre 200 et 1000. Le logiciel LabSolutions (version 5.60 SP2) est utilisé pour exploiter le système.

La quantification en moracénine B se fait grâce à la mesure de l'aire du pic d'un standard de moracénine B

obtenu par la demanderesse à partir d'un extrait racinaire de *Morus alba* résultant de la macération de 500 g de racines fraîches dans 1 litre d'une solution hydroéthanolique à 70/30 (éthanol/eau - v/v) pendant 48 heures. Brièvement, la fraction purifiée de moracénine B est obtenue par une méthode de séparation de phase suivie d'une HPLC préparative. Puis, la fraction est caractérisée par HPLC suivie d'une RMN. Le standard de moracénine B est préparé à la concentration de 100 mg/L dans un mélange DMSO/eau selon le ratio 70/30 et acidifié au pH 4 avec de l'acide chlorhydrique. Les teneurs des autres composés (moracénine A, kuwanone C, wittiorumine F et mulberrofurane T) sont exprimées en équivalents de moracénine B dans chaque extrait. La teneur est calculée selon la formule suivante pour un composé :

Teneur en composé en mg/L =

$$100 \text{ x} \frac{\text{(aire du pic correspondant au composé de l'extrait)}}{\text{(aire du pic correspondant à la moracénine B de la solution standard)}} \; [mg/L]$$

La moracénine B est utilisée comme standard de quantification pour les différents composés (moracénine A, moracénine B, kuwanone C, wittiorumine F et mulberrofurane T) car ces différents composés appartiennent à la même famille de molécules.

Les figures 1A, 1B et 1C montrent les chromatogrammes des extraits préparés à partir de racines de *M. alba non* stimulées (figure 1A) ou stimulées (figure 1B) et dilués 5 fois avant injection. La teneur en extrait sec (ES) est évaluée à 2,6 g/L pour les extraits de *M. alba* réalisés à partir de racines stimulées et de 2,3 g/L pour les extraits de *M. alba* réalisés à partir de racines non stimulées.

Les teneurs en composés sont indiquées dans le tableau 1 ci-dessous.

**Tableau 1**

| Composé | Extrait de *M. alba* | Teneur en eq. en moracénine B (mg/L) pour 20 mg de racines sèches broyées | | Teneur en eq. en moracénine B en % d'ES |
|---|---|---|---|---|
| | | Valeur | Ecart type, n = 3 | Valeur |
| moracénine B | Racines stimulées | 126,1 | 3,1 | 4,8 |
| | Racines non stimulées | 53,5 | 1,2 | 2,3 |
| kuwanone C | Racines stimulées | 83,2 | 1,6 | 3,2 |
| | Racines non stimulées | 3,4 | 0,1 | 0,15 |
| moracénine A | Racines stimulées | 73,8 | 1,1 | 2,8 |
| | Racines non stimulées | 28,5 | 0,7 | 1,2 |
| wittiorumine F | Racines stimulées | 22,8 | 0,6 | 0,9 |
| | Racines non stimulées | 18,1 | 0,7 | 0,8 |
| mulberrofurane T | Racines stimulées | 13 | 0,5 | 0,5 |
| | Racines non stimulées | 9,3 | 0,4 | 0,4 |

Dans le présent exemple, la stimulation des racines de *M. alba* par carence azotée permet une augmentation de la teneur dans l'extrait racinaire (en mg/L) :

- de 136% en moracénine B

- de 2358% en kuwanone C
- de 159% en moracénine A
- de 26% de wittiorumine F
- de 39% de mulberrofurane T

Afin de vérifier que l'extrait racinaire *de M. alba* selon l'invention est riche en polyphénols prenylés, c'est-à-dire que l'extrait racinaire selon l'invention comprend une quantité supérieure en moracénine B, moracénine A, kuwanone C, wittiorumine F et mulberrofurane T par rapport à un extrait correspondant issu d'une écorce de racine de plantes du genre *Morus* pouvant être trouvé dans le commerce, le profil phytochimique d'un extrait d'écorce de racine séchées de *M. alba* (encore appelé *Cortex Mori Albae Radicis* ou Sang Bai Pi) a été réalisé. Ces écorces (origine Chine, commandées par la demanderesse en 2015) se présentent sous la forme de copeaux épais de couleur jaune pâle. Les écorces sont broyées pour être réduites à l'état de poudre, avec un broyeur à billes (WWR star-beater) pendant 5 minutes à la vitesse de 20 battements par seconde et encore 5 minutes à la vitesse de 30 battements par seconde. 20 mg de Sang Bai Pi broyés sont mis à macérés dans 1 mL d'éthanol pur sous agitation (à l'aide d'un vortex) à température ambiante pendant 30 minutes. Les échantillons sont centrifugés à 21000 g pendant 10 minutes afin de séparer la matière solide. Les surnageants sont récupérés et dilués 5 fois dans de l'éthanol pur pour être injectés. La figure 1C montre le chromatogramme de l'extrait préparé à partir d'écorce. La teneur en extrait sec (ES) dans les extraits a été évaluée à 0,63 g/L.
La quantification des polyphénols prenylés est réalisé selon le protocole décrit ci-dessous et les valeurs sont indiquées dans le tableau 2 ci-dessous :

**Tableau 2**

| Composé | Teneur en eq. en moracénine B (mg/L) pour 20 mg d'écorce de racines séchées de *M. alba* | | Teneur en eq. en moracénine B en % d'extrait sec |
|---|---|---|---|
| | Valeur | Ecart type, n = 3 | Valeur |
| moracénine B | 11,74 | 0,34 | 1,86 |
| moracénine A | 0,16 | 0,04 | 0,025 |
| Kuwanone C | 3,86 | 0,22 | 0,61 |
| wittiorumine F | 0 | 0 | 0 |
| mulberrofurane T | 0 | 0 | 0 |

L'extrait selon l'invention se distingue par la présence de wittiorumine F et de mulberrofurane T qui ne sont pas détectés dans l'extrait d'écorce commerciale, par des teneurs très supérieures en moracénine A et B et par des teneurs supérieures en kuwanone C pour les racines stimulées.
La présence de wittiorumine F et de mulberrofurane T a été confirmée dans des extraits racinaires de *Morus nigra* préparés selon le même protocole utilisé pour l'obtention des extraits de *Morus alba* et décrit dans le présent exemple. La teneur en extrait sec (ES) est évaluée à 2,67 g/L pour l'extrait de *M. nigra* réalisé à partir de racines stimulées pendant 4 semaines et de 3,16 g/L pour l'extrait de *M. nigra* réalisés à partir de racines non stimulées.
La quantification des polyphénols prenylés est réalisé selon le protocole décrit ci-dessous et les valeurs (n=1) sont indiquées dans le tableau 3 ci-dessous :

**Tableau 3**

| Composé | Extrait de *M.* nigra | Teneur en eq. en moracénine B (mg/L) pour 20 mg de racines séchées broyées | Teneur en eq. en moracénine B en % d'ES |
|---|---|---|---|
| wittiorumine F | Racines stimulées | 25 | 0,93 |
| | Racines non stimulées | 8 | 0,25 |

(suite)

| Composé | Extrait de *M.* nigra | Teneur en eq. en moracénine B (mg/L) pour 20 mg de racines séchées broyées | Teneur en eq. en moracénine B en % d'ES |
|---|---|---|---|
| mulberrofurane T | Racines stimulées | 13 | 0,48 |
| | Racines non stimulées | 5 | 0,16 |

Dans le présent exemple, la stimulation des racines de *M. nigra* par carence azotée permet une augmentation de la teneur dans l'extrait racinaire (en mg/L) :

- de 212% de wittiorumine F
- de 160% de mulberrofurane T

**EXEMPLE 2 : ACTIVITE INHIBITRICE ANTI-TYROSINASE D'UN EXTRAIT RACINAIRE DE MORUS ALBA.**

**2.1 Test d'activité inhibitrice anti-tyrosinase sur racines de M. *alba* non stimulées**

Des plants de *Morus alba* sont cultivées en aéroponie pendant 8 semaines avec un milieu de culture 15/10/30 (N/P/K) et à une électroconductivité comprise entre 1,0 et 1,2 mS/cm. Des racines sont coupées, séchées, broyées et mises à macérer dans de l'éthanol pur à un ratio de 1 mL d'éthanol pour 20 mg de matière sèche selon le protocole décrit à l'exemple 1. L'échantillon est centrifugé à 21000 g pendant 10 minutes afin de séparer la matière solide. L'activité inhibitrice anti-tyrosinase du surnageant est mesurée en utilisant la L-tyrosine comme substrat. La tyrosinase catalyse la conversion de la L-tyrosine en dopaquinone qui est spontanément convertit en dopachrome. Ce dernier composé absorbe fortement dans la gamme visible du spectre électromagnétique (à 475 nm). L'activité de la tyrosinase est mesurée en suivant la conversion enzymatique de la L-tyrosine incolore en dopachrome orange à l'aide du lecteur de microplaques.

L'activité anti-tyrosinase du surnageant est réalisée en mélangeant les composants suivants : 150 $\mu$L de la solution de L-tyrosine (1,5 mM) dans du tampon phosphate de sodium 50 mM, pH 6,8; 40 $\mu$L du surnageant dilué de 2 à 800 fois dans de l'éthanol est testée en tant que solution d'inhibiteur. La conversion enzymatique est initialisée en ajoutant un volume de 10 $\mu$L de tyrosinase de champignon (0,2 mg / ml dans un tampon phosphate de sodium 50 mM, pH 6,8). L'expérience de «contrôle» a été menée de la même manière en remplaçant le surnageant par de l'éthanol pur.

La figure 2 montre le pourcentage d'activité tyrosinase en fonction du facteur de dilution du surnageant. Pour des dilutions comprises entre 2 et 16 on observe une inhibition quasi-totale de l'activité. La concentration inhibitrice médiane CI50 a été évaluée pour le facteur de dilution de 150, qui correspond à un extrait préparé à partir de 0,133 mg de matière sèche dans 1 mL d'éthanol pur. A partir des données du tableau 1 (exemple 1, racines de *M. alba* non stimulées) sont calculées les teneurs en équivalent en moracénine B (mg/L) des 5 polyphénols prenylés contenus dans un extrait préparé à partir de 133 mg de racine sèches de *M. alba* dans 1 L d'éthanol, soit :

- 0,36 mg/L de moracénine B
- 0,02 mg/L de kuwanone C exprimé en équivalent moracénine B
- 0,19 mg/L de moracénine A exprimé en équivalent moracénine B
- 0,12 mg/ L de de wittiorumine F exprimé en équivalent moracénine B
- 0,06 mg/L de mulberrofurane T exprimé en équivalent moracénine B

**2.2 Comparaison de l'activité inhibitrice anti-tyrosinase sur racines de *M. alba* non stimulées et stimulées par carence azotée.**

Deux extraits racinaires de *Morus alba* sont préparés selon le protocole décrit à l'exemple 1. La mesure de l'activité inhibitrice anti-tyrosinase est réalisée selon le protocole décrit à l'exemple 2.1 avec 40 $\mu$L de chacun des deux extraits dilués de 100 fois ou 200 fois dans de l'éthanol pur.

Le tableau 4 ci-dessous présente le pourcentage d'inhibition de l'activité tyrosinase pour chacun des extraits racinaires.

**Tableau 4**

| Facteur de dilution | Type racines *M. alba* | Pourcentage inhibition activité tyrosinase |
|---|---|---|
| 100 | stimulées | 86% |
| | non stimulées | 70% |
| 200 | stimulées | 77% |
| | non stimulées | 49% |

L'extrait préparé à partir de racines stimulées par carence azotée inhibe plus fortement l'activité tyrosinase (86% et 77%) que l'extrait préparé à partir de racines non stimulées (70% et 49%).

**2.3 Evaluation de l'affinité des composés présents dans un extrait racinaire de M. *alba* pour la cible tyrosinase** : **Test d'affinité par Target Binding®**

Un extrait racinaire de *Morus alba* a été préparé selon le protocole décrit à l'exemple 2.1.

La méthode de Target Binding®, décrite dans la demande de brevet FR1670545 correspond à un procédé de détermination de l'affinité entre des ligands et une cible. Elle repose sur le principe de la mise en contact en phase liquide des ligands et de la cible pour que les ligands qui ont le plus d'affinité avec la cible s'y lient, de l'élimination des ligands non fixés, de la séparation des complexes pour récupérer les ligands qui étaient fixés, puis de la quantification de ces derniers.

Dans une première étape du procédé, l'extrait et la cible sont mélangés. Les composés de l'extrait se font concurrence pour accéder aux sites de liaison de la cible. Les composés les plus forts déplacent les plus faibles et sont retenus de manière plus efficace par la cible.

Par la suite, on procède aux étapes suivantes:

i) séparation des complexes ligand-cible du mélange ;
ii) élimination des composés non liés ;
iii) dénaturation des complexes ligand-cible pour relâcher les ligands ;
iv) élimination de la cible dénaturée par précipitation et centrifugation ;
v) analyse du surnageant : comparaison des signaux analytiques des ligands du surnageant avec les signaux analytiques des ligands de l'échantillon.

La cible, une solution de tyrosinase de champignon à 1,5 mg/mL est préparée en dissolvant de la poudre de l'enzyme lyophilisée dans 50 mM d'acétate d'ammonium, avec un pH de 7.0. L'échantillon à analyser correspondant au surnageant de l'extrait racinaire obtenu selon l'exemple 2.1. Il peut être éventuellement centrifugé une nouvelle fois avant utilisation à 21000 g pendant 15 min afin d'enlever toutes traces de particules solides. Un échantillon de référence est préparé également en diluant l'échantillon à analyser 10 fois dans une solution d'eau et d'un quart de volume d'acétonitrile.

Les étapes de la méthode sont les suivantes :

1. Étape de préconditionnement - Des puits à membrane filtrante sont préconditionnées avec un volume de 500 µl d'une solution à 85% d'acétate d'ammonium et 15% d'éthanol qui est passé à travers la membrane avec une centrifugation de 14000 g pendant 60 secondes.
2. Étape de liaison - La préparation de tyrosinase (1,5 mg/mL, 92,5 µL) est mélangée avec 7,5 µl de l'échantillon à analyser pendant 5 min dans un tube Eppendorf en plastique.
3. Étape de lavage - le mélange de l'enzyme et de l'échantillon à analyser est déposé dans un puits préconditionné à l'étape de préconditionnement, puis centrifugé à 14000 g jusqu'à l'élimination de l'essentiel du solvant. Les complexes enzyme-ligands restent à la surface de la membrane du filtre puis sont à nouveau suspendus dans un volume de 100 µL de la même solution à 85% d'acétate d'ammonium et 15% d'éthanol et centrifugé à 14000 g jusqu'à élimination du solvant. Cette procédure est répétée trois fois et élimine tous les composés qui ne sont pas liés à l'enzyme.
4. Étape de séparation - Les complexes enzyme-ligands retenus dans le filtre sont à nouveau suspendus dans un volume de 10 µL d'eau ultra-pure puis mélangés avec un volume de 40 µL d'acétonitrile pour dénaturer et précipiter l'enzyme et, en même temps libérer les ligands. Le mélange est centrifugé à 21000 g pendant 10 min pour séparer le précipité du surnageant.

5. Étape d'analyse - Ledit surnageant est analysé par chromatographie en phase liquide couplée à une spectrométrie de masse, connue également par l'acronyme UPLC-MS. L'échantillon de référence est analysé également de la même manière, en utilisant les mêmes conditions d'analyse.

L'analyse UPLC-MS est réalisée selon le protocole décrit à l'exemple 1.

Les chromatogrammes obtenus sont présentés dans les figures 3A et 3B et représentent une intensité de signal en fonction du temps de rétention dans une colonne. Le signal présente différents pics d'intensité variable. Chaque pic est associé à l'un des ligands présent dans l'extrait analysé. La quantité associée à chaque ligand correspond à l'aire du pic.

Le chromatogramme (figure 3A) de l'échantillon de référence permet d'identifier les composés majoritaires présents dans l'extrait racinaire à savoir :

1 - moracénine B ;
2 - kuwanone C ;
3 - moracénine A ;
4 - wittiorumine F ;
5 - mulberrofurane T;

La comparaison de la taille des aires des pics du chromatogramme de la figure 3B obtenu après liaison des composés de l'échantillon à analyser sur la tyrosinase permet une évaluation grossière de l'ordre d'affinité des composés de l'extrait racinaire selon l'exemple 2.1 pour la tyrosinase : l'aire du pic est supérieure pour la mulberrofurane T et la wittiorumine F montrant une affinité plus importante pour ces deux composés.

Afin de valider ce résultat, les affinités relatives sont calculées selon la méthode décrite dans la demande de brevet FR1670545.

On calcule pour chaque ligand un rapport d'affinité RACEi tel que :

$$RA_{CEi} = \frac{NF_{TB,CEi}}{NF_{eq,CEi}}$$

avec :

$$NF_{eq,CEi} = \frac{IS_{eq,CEi}}{IS_{eq,REF}}$$

et

$$NF_{TB,CEi} = \frac{IS_{TB,CEi}}{IS_{TB,REF}}$$

où :

NF est un facteur de normalisation ;

ISeq,CEi est l'intensité du signal analytique pour le ligand CEi dans l'échantillon de référence;

ISeq,REF est l'intensité du signal analytique pour le ligand de référence REF (ici la moracénine B) dans l'échantillon de référence;

ISTB,CEi est l'intensité du signal analytique pour le ligand CEi dans la solution traitée (l'échantillon à analyser après les étapes 2 à 4 de liaison et de séparation des ligands) des ligands détachés;

ISTB,REF est l'intensité du signal analytique pour le ligand de référence REF (ici la moracénine B) dans la solution traitée (l'échantillon à analyser après les étapes 2 à 4 de liaison et de séparation des ligands) des ligands détachés;

RA, CEi est le rapport d'affinité relative (RA) représentant une affinité du ligand CEi pour la cible. La valeur du rapport est croissante avec l'affinité.

**Tableau 5** : Affinité relative de la moracénine B (considéré comme le ligand de référence), la moracénine A, la kuwanone C, la wittiorumine F et la mulberrofurane T présents dans l'extrait racinaire de *M. alba* selon l'exemple 2.1 vis-à-vis de la tyrosinase.

| Affinité relative (RA) pour la tyrosinase | | | | |
|---|---|---|---|---|
| moracénine B (référence) | kuwanone C | moracénine A | wittiorumine F | mulberrofurane T |
| 1,0 | 4,0 | 5,1 | 15,5 | 36,2 |

Les affinités relatives confirment les résultats basés sur l'évaluation des aires des pics, à savoir une affinité pour la tyrosinase supérieure pour la mulberrofurane T et la wittiorumine F par rapport aux autres composés évalués.

**EXEMPLE 3** : **ACTIVITE INHIBITRICE ANTI-COLLAGENASE D'UN EXTRAIT RACINAIRE DE MORUS ALBA.**

**3.1 Comparaison de l'activité inhibitrice anti-collagénase sur racines de *M. alba* non stimulées et stimulées par carence azotée.**

Deux extraits racinaires de *Morus alba* sont préparés selon le protocole décrit à l'exemple 1. La mesure de l'activité inhibitrice anti-collagénase est réalisée selon le protocole suivant. L'activité inhibitrice anti-collagénase des deux extraits est mesurée en utilisant le FALGPA (N-[3-(2-Furyl)acryloyl]-Leu-Gly-Pro-Ala) comme substrat. La collagénase catalyse la conversion du FALGPA en FAL (N-(3[2-Furyl]acryloyl)-Leu) et Gly-Pro-Ala. La dégradation du FALGPA est à l'origine d'une diminution de l'absorption du mélange réactionnel à 345 nm. L'activité de la collagénase est mesurée en suivant la conversion enzymatique de la FALGPA en FAL + Gly-Pro-Ala à l'aide du spectrophotomètre UV VIS.

Le mélange réactionnel est le suivant : 510 μL de la solution de FALGPA (1,5 mM) dans du tampon Tris-HCl 50 mM, CaCl2 10 mM, NaCl 400 mM pH 7,5; 30 μL de chacun des deux extraits dilué 5 fois dans de l'éthanol pur est testée en tant que solution d'inhibiteur. La conversion enzymatique est initialisée en ajoutant un volume de 10 μL de collagénase issue de *Clostridium Histolyticum* (0,8 mg / mL dans un tampon Tris-HCl 50 mM, CaCl2 10 mM, NaCl 400 mM, pH 7,5, L'expérience de «contrôle» a été menée de la même manière en remplaçant le surnageant par de l'éthanol pur.

L'extrait préparé à partir de racines stimulées par carence azotée inhibe plus fortement l'activité collagénase (94%) que l'extrait préparé à partir de racines non stimulées (83%).

**3.2 Evaluation de l'affinité des composés présents dans un extrait racinaire de M. *alba* pour la cible collagénase** : **Test d'affinité par Target Binding®**

Un extrait racinaire de *Morus alba* a été préparé selon le protocole décrit à l'exemple 2.1. La méthode de Target Binding® utilisé est celle de l'exemple 2.3 qui est décrite dans la demande de brevet FR1670545. Le protocole expérimental est le suivant :

La cible, une solution de collagénase issue de *Clostridium histolyticum* à 6,25 mg/mL est préparée en dissolvant de la poudre de l'enzyme lyophilisée dans 50 mM de tampon phosphate salin, avec un pH de 7.5. L'échantillon à analyser correspondant au surnageant de l'extrait racinaire obtenu selon l'exemple 2.1. Il peut être éventuellement centrifugé une nouvelle fois avant utilisation à 21000 g pendant 15 min afin d'enlever toutes traces de particules solides. Un échantillon de référence est préparé également en diluant l'échantillon à analyser 10 fois dans une solution d'eau et d'un quart de volume d'acétonitrile.

Les étapes de la méthode sont les suivantes :

1.2. Analyse de l'échantillon

1. Étape de préconditionnement - Des puits à membrane filtrante sont préconditionnées avec un volume de 500 μl d'un tampon phosphate salin qui est passé à travers la membrane avec une centrifugation de 14000 g pendant 60 secondes.
2. Étape de liaison - Un volume de 92,5 μl de la solution issue de l'étape de prétraitement est mélangé avec 7,5 μl de l'échantillon à analyser pendant 10 min dans un tube Eppendorf en plastique.

3. Étape de lavage - Le mélange de l'enzyme et de l'échantillon à analyser est déposé dans un puits préconditionné à l'étape de préconditionnement, puis centrifugé à 14000 g jusqu'à l'élimination de l'essentiel du solvant. Les complexes enzyme-ligands restent à la surface de la membrane du filtre puis sont à nouveau suspendus dans un volume de 100 µL de la même solution du tampon phosphate salin et centrifugé à 14000 g jusqu'à élimination du solvant. Cette procédure est répétée trois fois et élimine tous les composés qui ne sont pas liés à l'enzyme.

4. Étape de séparation - Les complexes enzyme-ligands retenus dans le filtre sont à nouveau suspendus dans un volume de 10 µL d'eau ultra-pure puis mélangés avec un volume de 30 µL d'acétonitrile pour dénaturer et précipiter l'enzyme et, en même temps libérer les ligands. Le mélange est centrifugé à 21000 g pendant 10 min pour séparer le précipité du surnageant.

5. Étape d'analyse - Ledit surnageant est analysé par chromatographie en phase liquide couplée à une spectrométrie de masse. L'échantillon de référence est analysé également de la même manière, en utilisant les mêmes conditions d'analyse.

L'analyse UPLC-MS est réalisée selon le protocole décrit à l'exemple 1.

Le chromatogramme (figure 4A) de l'échantillon de référence permet d'identifier les composés majoritaires présents dans l'extrait racinaire selon l'exemple 2.1, à savoir :

1 - moracénine B ;
2 - kuwanone C ;
3 - moracénine A ;
4 - wittiorumine F ;
5 - mulberrofurane T.

La taille des aires des pics du chromatogramme de la figure 4B obtenu après liaison des composés de l'extrait sur la collagénase permet de monter une affinité semblable de la mulberrofurane T, de la wittiorumine F et des moracénine A et B pour la collagénase.

Afin de valider ce résultat, les affinités relatives sont calculées selon la méthode de l'exemple 2.3 et décrite dans la demande de brevet FR1670545.

**Tableau 6 :** Affinité relative de la moracénine B (considéré comme le ligand de référence), la moracénine A, la kuwanone C, la wittiorumine F et la mulberrofurane T présents dans l'extrait racinaire de *M. alba* vis-à-vis de la collagénase.

| Affinité relative (RA) pour la collagénase | | | | |
|---|---|---|---|---|
| moracénine B (référence) | kuwanone C | moracénine A | wittiorumine F | mulberrofurane T |
| 1,0 | 0,9 | 1,1 | 1,1 | 1,1 |

Les affinités relatives vis-à-vis de la collagénase sont semblables pour les 5 composés.

## EXEMPLE 4 : ACTIVITE INHIBITRICE ANTI-TYRONISASE DE MOLECULES PURIFIEES A PARTIR DE RACINES DE *MORUS ALBA* ET DE *MORUS NIGRA*

La wittiorumine F est purifiée à partir d'un extrait racinaire de *Morus nigra* cultivées en aéroponie, puis stimulées par un stress azoté via l'utilisation d'un milieu nutritif sans azote (milieu 0/15/40) pendant 2 à 6 semaines. 500 g racines fraîches sont mises à macérer dans 1 litre d'une solution hydroéthanolique à 70/30 (éthanol/eau - v/v) pendant 48 heures Le pH du milieu de macération n'est pas ajusté. Brièvement, la fraction purifiée de wittiorumine F est obtenue par une HPLC préparative et caractérisée par RMN. La moracénine A, la moracénine B et la kuwanone C ont été purifiées à partir d'un extrait racinaire de *Morus alba* selon le protocole décrit à l'exemple 1. La mesure de l'activité inhibitrice anti-tyrosinase est réalisée selon le protocole décrit à l'exemple 2.1.

L'activité inhibitrice de la tyrosinase a été testée pour les 4 composés à 3 concentrations et les résultats sont présentés dans le tableau ci-dessous :

**Tableau 7 :** Pourcentage de l'activité inhibitrice de la tyrosinase pour les 4 composés aux concentrations de 2,5 μM, 5 μM et 20 μM.

| | Concentration 2,5 μM | | Concentration 5 μM | | Concentration 20 μM | |
|---|---|---|---|---|---|---|
| Composé | % inhibition | concentra tion mg/L | % inhibition | concentra tion mg/L | % inhibition | concentra tion mg/L |
| Moracé nine A | 18,9 % | 1,9 | 27,7 % | 3,8 | 59,0 % | 15,2 |
| Moracé nine B | 22,6 % | 1,7 | 33,5 % | 3,5 | 69,7 % | 13,9 |
| Kuwano ne C | 24,7 % | 1,1 | 37,7 % | 2,1 | 72,7 % | 8,4 |
| Wittioru mine F | 35,7 % | 1,6 | 52,9 % | 3,2 | 75,1 % | 13 |

La wittiorumine F présente une meilleure activité inhibitrice, suivie de la kuwanone C, puis de la moracénine B et A. Ce résultat est conforme à l'exemple 2.3 montrant une affinité plus grande de la wittiorumine F pour la cible tyrosinase vis-à-vis des 3 flavonoïdes prenylés.

Dans l'exemple 2.1, la concentration inhibitrice médiane CI50 de la tyrosinase a été mesurée pour un extrait préparé à partir de 133 mg de racines sèches de *Morus alba* dans 1 L d'éthanol pur, correspondant un total de 0,69 mg/L de moracénine B et A, de kuwanone C et de wittiorumine F exprimé en équivalent moracénine B. Pour la wittiorumine F, qui est le composé le plus inhibiteur, une inhibition de 52,9% est mesurée à une concentration de 3,2 mg/L. Cette comparaison montre une activité inhibitrice plus forte pour l'extrait racinaire que pour les composés isolés.

**EXEMPLE 5** : **EXTRAIT RACINAIRE DE *MORUS ALBA* PREPARE A PARTIR D'UN SOLVANT GLYCOLE.**

Un extrait racinaire de *Morus alba* riche en polyphénols prenylés est obtenu selon le procédé suivant :

- Culture de *Morus alba* en aéroponie avec une solution nutritive définie, de composition N/P/K correspondant à 15/10/30 et à une électroconductivité comprise entre 1,0 et 1,2 mS/cm,
- Stimulation de la plante pendant une durée de 2 à 4 semaines par un stress azoté par l'utilisation d'une solution nutritive N/P/K comprenant : moins de 6% d'azote, 15% de phosphore et 40% de potassium, et d'une électroconductivité de 0,6 à 0,8 mS/cm,
- Extraction des composés d'intérêt par macération des racines coupées, séchées et broyées avec un broyeur orbital à billes (Fritsch - PULVERISETTE 6), pendant une durée comprise entre 5 minutes à 3 heures dans un mélange propane-1,3-diol/eau selon un ratio compris entre 85/15 et 70/30, à température ambiante et à un pH compris entre 3,5 et 4,5. La macération se déroule à température ambiante sous agitation. Le ratio matière sèche (MS) / solvant utilisé pour la macération est de 50 g MS / L de solvant. La macération se déroule dans un volume de 30 mL de solvant.

Les teneurs en moracénine B ainsi qu'en moracénine A, kuwanone C, wittiorumine F et mulberrofurane T exprimé en équivalent moracénine B dans l'extrait racinaire de *Morus alba* en fonction des durées de macération, évaluées selon la méthode décrite à l'exemple 1, sont indiquées dans la figure 5.

Les extraits ainsi obtenus présentent les caractéristiques suivantes :

- Teneur en extrait sec : 7,1 g/L à 10,2 g/L
- Teneur en moracénine B : 446 mg/L à 512 mg/L (soit 4,9% à 6,3% de l'extrait sec)
- Teneur en moracénine A exprimé en équivalent moracénine B: 162 mg/L à 182 mg/L (soit 1,7% à 2,3% de l'extrait sec)
- Teneur en kuwanone C exprimé en équivalent moracénine B: 184 mg/L à 196 mg/L (soit 1,8% à 2,7% de l'extrait sec)
- Teneur en wittiorumine F exprimé en équivalent moracénine B: 34 mg/L à 44,5 mg/L (soit 0,4% à 0,5% de l'extrait sec)
- Teneur en mulberrofurane T exprimé en équivalent moracénine B: 27 mg/L à 29 mg/L (soit 0,38% à 0,41% de l'extrait sec)
- pH : 6 +/- 1

Deux autres extraits racinaires de *Morus alba* riche en polyphénols prenylés sont obtenus selon le procédé suivant :
Les étapes de culture et de stimulation de *Morus alba* sont réalisés selon le protocole décrit dans ci-dessus dans le

présent exemple 1.

L'extraction des composés d'intérêt est réalisée par macération des racines coupées, séchées et broyées (avec un broyeur à couteaux), pendant une durée de 2 heures dans un mélange propane-1,3-diol/eau selon un ratio compris entre 85/15 et 70/30, à température ambiante et à un pH de 4 ou à un pH non ajusté. La macération se déroule à température ambiante sous agitation à l'aide d'un plateau d'agitation orbital. Le ratio matière sèche (MS) / solvant utilisé pour la macération est de 50 g MS / L de solvant. La macération se déroule dans un volume de 500 mL de solvant.

Les teneurs en moracénine B ainsi qu'en moracénine A, kuwanone C, wittiorumine F et mulberrofurane T exprimé en équivalent moracénine B sont évaluées selon la méthode décrite à l'exemple 1 et sont présentées dans le Tableau 8 ci-dessous :

**Tableau 8** : Teneurs en extrait sec, moracénine A ainsi qu'en moracénine B kuwanone C, wittiorumine F et mulberrofurane T exprimé en équivalent moracénine B et indiqués en mg/L ou en % d'extrait sec (ES) pour deux extraits racinaires de *Morus alba* obtenus après une macération de deux heures à un pH de 4 ou à un pH non ajusté.

| | Teneur en extrait sec (ES) | moracénine A | | moracénine B | | kuwanone C | | wittiorumine F | | mulberrofurane T | |
|---|---|---|---|---|---|---|---|---|---|---|---|
| | g/L | mg / L | % d'ES | mg / L | % d'ES | mg / L | % d'ES | mg / L | % d'ES | mg / L | % d'ES |
| pH=4 | 5 | 114 | 2,3 | 395 | 7,9 | 106 | 2,1 | 20 | 0,4 | 16 | 0,31 |
| pH non ajusté | 5,1 | 122 | 2,4 | 421 | 8,25 | 112 | 2,2 | 22 | 0,42 | 17 | 0,33 |

**EXEMPLE 6** : **ACTIVITE INHIBITRICE ANTI-HYALURONIDASE D'UN EXTRAIT RACINAIRE DE MORUS ALBA.**

L'acide hyaluronique (AH) est un glycoaminoglycane qui compose la matrice extracellulaire. Il est produit par les HAS (Hyaluronic Acid Synthases) et comble les espaces intercellulaires de la peau afin de maintenir la cohésion des tissus et assurer leur hydratation en retenant les molécules d'eau. L'hyaluronidase catalyse l'hydrolyse de l'AH. Avec l'âge ou suite à des expositions répétées aux UV, l'expression de cette enzyme augmente, entraînant une diminution de la quantité d'acide hyaluronique dans la peau. Il existe donc un réel intérêt à inhiber cette enzyme dans le but de prévenir ou retarder l'apparition des effets du vieillissement (effet anti-âge).

**6.1 Test d'activité inhibitrice anti-hyaluronidase sur racines de M. *alba* stimulées**

Un extrait racinaire de *Morus Alba* a été préparé selon le protocole décrit à l'exemple 5. La hyaluronidase catalyse l'hydrolyse des acides hyaluroniques en monosaccharides, disaccharides et oligosaccharides courts. La mesure de l'activité inhibitrice anti-hyaluronidase est réalisée en utilisant l'acide hyaluronique (AH) comme substrat afin de suivre sa diminution en cours de la réaction enzymatique, la pente de diminution de la concentration en AH au cours de la réaction étant proportionnelle à l'activité enzymatique.

La concentration en AH est mesurée selon le principe suivant : En présence d'une solution acide d'albumine de sérum bovin, l'AH précipite. Ainsi, en cas de présence d'AH, un précipité est formé et en cas d'hydrolyse complète de l'AH, aucun précipité n'est observé. La quantité de précipitation est donc proportionnelle à la teneur en AH.

Le mélange réactionnel (MR) est le suivant : solution d'acide hyaluronique [0,03% (w/v)] dans le tampon phosphate, 300 mM, pH 5,35 : 187,5 μL ; le tampon phosphate, 50 mM, pH 7 : 52,5 μL ; la solution contenant l'extrait ou le solvant (blanc) : 10 μL. La conversion enzymatique est initialisée en ajoutant un volume de 250 μL de hyaluronidase provenant de testicules bovins (type IV-S) dans le tampon phosphate, 50 mM, pH 7 (20 μg/mL, l'activité entre 15 et 60 U/mL). Ce mélange est incubé à 37°C sous agitation (à l'aide d'un vortex) pendant toute la durée de la réaction enzymatique. Pour suivre la dégradation enzymatique de l'AH, un volume de 50 μl du MR est prélevé toutes les 15 minutes, mélangé avec un volume de 250 μL de la solution de BSA acide (solution acide d'albumine de sérum bovin : 24 mM acétate de sodium, 79 mM acide acétique et 0,1% (w/v) albumine de sérum bovin, pH 3,75) puis incubé pendant 10 min à température ambiante. La quantité de précipitation formée est ensuite mesurée par lecteur de plaque (diffusion de la lumière à 600 nm).

L'extrait racinaire de *Morus alba* préparé selon l'exemple 5 inhibe fortement l'activité de la hyaluronidase : pour 1 % (v/v) d'extrait on observe une inhibition de 100%, à 0,5 % (v/v) d'extrait on observe une inhibition de 55,5% et à 0,1 % (v/v) d'extrait on observe une inhibition de 34,5%.

La mesure de l'activité inhibitrice anti-hyaluronidase de la moracénine A a été réalisée selon le même protocole que celui de l'exemple 5.

A une concentration de 10 μM, on mesure une inhibition de 55,1 %. Cette activité inhibitrice de la moracénine A est plus forte que celle de la quercétine, inhibiteur connu de la hyaluronidase (15,6% d'inhibition à une concentration de 10 μM).

**6.2 Evaluation de l'affinité des composés présents dans un extrait racinaire de M. *alba* non stimulées pour la cible hyaluronidase** : **Test d'affinité par Target Binding®**

Un extrait racinaire de *Morus Alba* a été préparé selon le protocole décrit à l'exemple 2.1. La méthode de Target Binding® utilisée est celle de l'exemple 2.3 qui est décrite dans la demande de brevet FR1670545. Le protocole expérimental est le suivant : La cible, une solution de hyaluronidase à 5 mg/mL est préparée en dissolvant de la poudre de l'enzyme lyophilisée dans 50 mM phosphate de sodium, avec un pH de 7.0. L'extrait correspondant au surnageant obtenu selon l'exemple 2.1. Il peut être éventuellement centrifugé une nouvelle fois avant utilisation à 21000 g pendant 15 min pour éliminer les particules solides avant utilisation. Un échantillon de référence est préparé également en diluant l'échantillon 10 fois dans une solution d'eau et d'un quart de volume d'acétonitrile.

Les étapes de la méthode sont les suivantes :

1. Étape de préconditionnement - Des puits à membrane filtrante sont préconditionnées avec un volume de 500 μl d'une solution de 50 mM phosphate de sodium, avec un pH de 7.0 qui est passé à travers la membrane avec une centrifugation de 14000 g pendant 60 secondes.
2. Étape de liaison - La préparation de hyaluronidase (5 mg/mL) est mélangée avec 7,5 μl de l'extrait racinaire pendant 5 min dans un tube d'Eppendorf en plastique.
3. Étape de lavage - le mélange de l'enzyme et de l'extrait racinaire est déposé dans un puits préconditionné à l'étape de préconditionnement, puis centrifugé à 14000 g jusqu'à l'élimination de l'essentiel du solvant. Les complexes enzyme-ligands restent à la surface de la membrane du filtre puis sont à nouveau suspendu dans un volume

de 100 µL de la même solution de 50 mM phosphate de sodium, avec un pH de 7.0 et centrifugé à 14000 g jusqu'à élimination du solvant. Cette procédure est répétée trois fois et élimine tous les composés qui ne sont pas liés à l'enzyme.

4. Étape de séparation - Les complexes enzyme-ligands retenus dans le filtre sont à nouveau suspendus dans un volume de 10 µL d'eau ultra-pure puis mélangés avec un volume de 40 µL d'acétonitrile pour dénaturer et précipiter l'enzyme et, en même temps libérer les ligands. Le mélange est centrifugé à 21000 g pendant 10 min pour séparer le précipité du surnageant.

5. Étape d'analyse - Ledit surnageant est analysé par chromatographie en phase liquide couplée à une spectrométrie de masse, connue également par l'acronyme UPLC-MS. L'échantillon de référence est analysé également de la même manière, en utilisant les mêmes conditions d'analyse.

L'analyse UPLC-MS est réalisée selon le protocole décrit à l'exemple 1.

Le chromatogramme (figure 6A) de l'échantillon de référence permet d'identifier les composés majoritaires présents dans l'extrait racinaire selon l'exemple 2.1, à savoir :

1 - moracénine B;
2 - kuwanone C;
3 - moracénine A;
4 - wittiorumine F;
5 - mulberrofurane T;

La taille des aires des pics du chromatogramme de la figure 6B obtenu après liaison des composés de l'extrait sur la collagénase permet de montrer une affinité semblable de la mulberrofurane T, de la wittiorumine F et des moracénine A et B pour la hyaluronidase.

Afin de valider ce résultat, les affinités relatives sont calculées selon la méthode de l'exemple 2.3 et décrite dans la demande de brevet FR1670545.

**Tableau 9** : Affinité relative de la moracénine B (considéré comme le ligand de référence), la moracénine A, la kuwanone C, la wittiorumine F et la mulberrofurane T présents dans l'extrait racinaire de *M. alba* vis-à-vis de la hyaluronidase.

| Affinité relative (RA) pour la hyaluronidase | | | | |
|---|---|---|---|---|
| Moracénine B (ref) | Kuwanone C | Moracénine A | Wittiorumine F | Mulberrofurane T |
| 1.0 | 1.2 | 1.9 | 2.1 | 2.7 |

Les affinités relatives vis-à-vis de la hyaluronidase sont semblables pour les 5 composés.

**EXEMPLE 7** : **CARACTERISATION D'UN EXTRAIT RACINAIRE DE *MORUS ALBA* VIS-A-VIS D'UN BENEFICE POUR LA PEAU - IDENTIFICATION DE CIBLES PAR ANALYSE DES MODIFICATIONS D'EXPRESSION DE GENES PAR QRT-PCR SUR CARTES TAQMAN**

L'étude consiste à mesurer les effets de l'extrait racinaire de *Morus alba* par qRT-PCR sur cartes TaqMan microfluidiques, d'une part, sur l'expression de 94 gènes impliqués dans la biologie du derme, le remodelage des tissus conjonctifs et le vieillissement (carte « Dermal Benefits » définie par la société StratiCELL) et, d'autre part, sur l'expression de 94 gènes impliqués dans les fonctions clés de l'épiderme, telles que la fonction barrière en lien direct avec l'hydratation, la réponse antioxydante, ou encore la pigmentation par les mélanocytes (carte « Epidermal Benefits » définie par la société StratiCELL). Le protocole a consisté à ajouter de l'extrait racinaire de *Morus alba* dans le milieu de culture de fibroblastes humains NHDFs (Normal Human Dermal Fibroblasts) en monocouche et d'épidermes humains mélanisés reconstitués (RHE/MEL/001), et, après 24 h, à analyser les différentes populations d'ARN pour identifier les gènes différentiellement exprimés par qRT-PCR.

Une étude préalable de cytotoxicité a permis de définir la concentration de travail de l'extrait racinaire de *Morus alba* pour l'étude d'expression génique. Le TGF-β1 (facteur de croissance transformant ou Transforming Growth Factor-beta-1) et la vitamine D3 (1α,25-dihydroxyvitamin D3), dont les effets sont documentés dans la littérature, ont été utilisés comme molécules de référence afin de valider les systèmes d'essai et la méthode d'analyse.

### 7.1 Matériels et méthodes

**• Extrait**

Les plantes *Morus alba* sont cultivées en aéroponie avec une stimulation par une carence azotée avec le milieu 0/15/40. Les racines fraîches sont coupées puis macérées à température ambiante pendant 48 heures dans une solution hydroéthanolique à 70/30 (éthanol/eau - v/v) à un ratio de 500 g de racines pour un litre de solvant et à un pH non ajusté. Ensuite l'extrait est filtré. Le solvant est éliminé en utilisant un évaporateur rotatif et la poudre séchée au dessiccateur. Un dosage de la moracénine B a été effectué sur l'extrait grâce à trois gammes en 4 points de moracénine B purifiée par la demanderesse selon le protocole décrit à l'exemple 1. Une solution à 1mM puis des dilutions jusqu'à 16 μM, ont été réalisées dans du DMSO 70%. L'analyse UPLC a été faite sur ces gammes. Les aires de pics correspondants à la moracénine B dans l'extrait ont ensuite été comparées aux aires de pics de la gamme. La teneur en moracénine B dans l'extrait correspond à 4% de l'extrait sec.

**• Culture Cellulaire**

La première partie de l'étude a été réalisée sur des fibroblastes de derme humains NHDFs (ATCC, CRL-2522, origine : prépuce) cultivés en monocouche en milieu DMEM (Invitrogen, 31885-049) contenant des antibiotiques (Pénicilline/Streptomycine, Invitrogen, 15140-122) mais ne contenant pas de sérum. Ces cellules ont été maintenues dans une atmosphère humide à 37°C contenant 5% de CO2.

La seconde partie de l'étude a été réalisée sur des épidermes reconstitués (StratiCELL®, RHE/MEL/001) contenant ou non des mélanocytes humains primaires NHEMs (Normal Human Epidermal Mélanocytes) provenant d'un donneur de phototype foncé (phototype IV à V) (Invitrogen, C2025C, lot n°439684). Les tissus ont été cultivés à l'interface air-liquide durant 14 jours dans un milieu de culture approprié, et une atmosphère humide à 37°C contenant 5% de CO2.

**• Détermination de la gamme de concentrations d'étude de l'extrait racinaire de *Morus alba* par une étude préliminaire de cytotoxicité**

Afin de déterminer la concentration d'analyse optimale pour l'extrait racinaire de *Morus alba,* une expérience préliminaire a été réalisée sur fibroblastes NHDFs, sur mélanocytes humains NHEMs et sur épidermes reconstitués. Les fibroblastes NHDFs ont effectué 30,1 et 30,7 doublements de population et les mélanocytes NHEMs ont été ensemencés en plaques 24 puits 24 h avant l'application des actifs.

Les épidermes reconstitués (RHE/001 ; lot CB0314/2) ont été transférés dans une plaque 12 puits avant d'être traités avec l'extrait.

L'extrait a été dilué dans le milieu de culture, puis ajouté, sans filtration préalable, dans le milieu de culture des fibroblastes humains NHDFs ne contenant pas de sérum, dans le milieu des mélanocytes humains primaires NHEMs, ou dans le milieu de culture des épidermes différenciés.

Cette étude a consisté à évaluer la viabilité des cellules et des épidermes au MTS (3-(4,5-dimethythiazol-2-yl)-5-(3-carboxy-methoxyphenyl)-2-(4-sulfophenyl)-2H-tetrazolium) (Promega,G3581), 24 heures après l'ajout d'extrait racinaire de *Morus alba* et ce, à 5 concentrations et 3 répétions (n=3) pour les fibroblastes NHDFs et les mélanocytes NHEMs, et à 2 concentrations et 3 répétitions (n=3) pour les épidermes reconstitués. Les 2 concentrations testées pour les épidermes reconstitués ont été choisies sur la base des résultats de cytotoxicité obtenus pour les mélanocytes NHEMs. Le SDS (sodium dodécyl sulfate) est toxique pour les cellules et a été utilisé en tant que contrôle positif afin de valider l'expérience.

Au terme de cette expérience, une concentration non cytotoxique a été définie afin de procéder à la mesure des modifications d'expression des gènes cibles.

Les concentrations suivantes, en μg d'extrait sec/mL, ont été testées :

- Fibroblastes NHDFs et les mélanocytes NHEMs: 0,16 μg/mL,0,8 μg/mL, 4 μg/mL, 20 μg/mL,100μg/mL ;
- Epidermes reconstitués : 20 μg ES/mL et 4 μg ES/mL

**• Analyse des modifications d'expression génique**

L'extrait a été ajouté, à une concentration choisie, dans le milieu de culture des fibroblastes humains NHDFs (ayant effectué 30,5 et 30,8 doublements de population) en absence de sérum ou dans le milieu de culture des épidermes différenciés mélanisés (RHE/MEL/001, lots CB0314/3 et CB0314/4), sans filtration préalable à la mise en contact des cellules/épidermes.

Des molécules de référence ont été étudiées en parallèle, à savoir, le TGF-β1 pour les fibroblastes NHDFs et

la vitamine D3 (VD3) pour les épidermes reconstitués.

• **Extraction de l'ARN total**

L'extraction des ARNs totaux a été réalisée à l'aide du kit RNeasy Mini (Qiagen, 74106). 24 h après l'ajout de l'extrait, les cellules ont été rincées au PBS et lysées dans le tampon de lyse *ad hoc,* alors que les épidermes ont été directement plongés dans ce tampon (des triples de culture ont été réalisés pour chaque condition). L'extraction et la purification des ARNs ont été réalisées selon les instructions du fournisseur. Les ARNs totaux ont ensuite été conservés à -80°C.

• **Qualification des ARNs par spectrophotométrie et électrophorèse capillaire**

La concentration des ARNs totaux a été déterminée par mesure spectrophotométrique. La qualité et l'intégrité des ARNs ont ensuite été vérifiées par électrophorèse capillaire (plate-forme Agilent Bioanalyzer 2100

- Agilent RNA 6000Nano Kit, 5067-1511).
- Quantification des ARN par mesure spectrophotométrique : un aliquot de chaque ARN a été dilué dans de l'eau RNase-free et sa concentration a été déterminée à l'aide d'un spectrophotomètre Ultrospec 1100 Pro (Amersham).
- Intégrité des ARN par électrophorèse capillaire sur Bioanalyseur Agilent : l'intégrité de l'ARN total a été évaluée par la visualisation des pics d'électrophorèse correspondant aux ARNs ribosomiaux. Pour les ARN totaux d'eucaryotes supérieurs, la taille des bandes ribosomales doit être de 1,9 kb pour le 18S-ARN et de 4,7 kb pour le 28S-ARN. L'intensité de la bande correspondant au 28S-ARN doit être supérieure à l'intensité de la bande correspondant au 18S-ARN. Des petites bandes diffuses représentant des ARNs de poids moléculaire plus faible (ARNt et l'ARN ribosomique 5S) peuvent être présentes. Lorsque l'ARN est dégradé, on observe un étalement des bandes de l'ARN ribosomal ainsi qu'un bruit de fond des ARN de poids moléculaire plus élevé.

• **Synthèse des ADNs complémentaires ou ADNc**

Les transcriptions inverses (RT) ont été réalisées à l'aide du kit « High Capacity RNA-to-cDNA Kit » (Applied Biosystems, 4387406). Pour la synthèse des ADNc, un mix a été préparé selon les instructions du fournisseur, avec 2 $\mu$g d'ARN total, le tampon *ad hoc* fourni dans le kit et l'enzyme transcriptase inverse. Cette réaction a été réalisée à 37°C pendant 1 heure, puis 5 minutes à 95°C et finalement les échantillons d'ADNc ont mis sur glace et stockés à - 20°C.

• **Validation des systèmes d'essai** - **qPCR en temps réel à l'aide de sondes fluorescentes de type TaqMan**

La méthode de qPCR en temps réel a été utilisée pour quantifier l'expression de différentes cibles spécifiques dans les populations d'ARNs issues des fibroblastes NHDF traités par le TGF-$\beta$1 (20ng/mL) ainsi que des épidermes mélanisés traités par la vitamine D3 (100nM) et par le solvant éthanol (EtOH 0,1%), utilisé pour solubiliser la VD3.

Les séquences cibles des gènes d'intérêt ont été amplifiées par PCR en utilisant les « TaqMan Gene Expression Assays » (Applied Biosystems). Ces kits comprennent une sonde TaqMan et 2 amorces spécifiques, qui ont été prémélangées à une concentration de 18 $\mu$M pour chaque amorce et de 5 $\mu$M pour la sonde. Ce mélange est concentré 20 fois. Les sondes TaqMan ont été greffées avec un fluorophore (FAM) à l'extrémité 5' de la séquence et avec un «quencher» de fluorescence en 3'.

Les PCRs ont été réalisées à l'aide du système 7900HT Fast Real-Time PCR (Applied Biosystems). Les réactions ont été réalisées dans un volume de 20 $\mu$L. Le mélange réactionnel contient 10 $\mu$L de TaqMan Fast Universal Master Mix (Applied Biosystems), 1 $\mu$L de TaqMan Gene Expression Assay et 5 $\mu$L d'eau RNase-free.

Dans chaque puits d'une microplaque 96 puits, 16 $\mu$L du mélange et 4 $\mu$L d'ADNc (4 ng) ont été ajoutés. A des fins de normalisation, des mélanges réactionnels avec des sondes et amorces correspondant à la glycéraldehyde-3-phosphate déshydrogénase (*GAPDH*) pour les fibroblastes et à la $\beta$2-microglobuline (*B2M*) pour les épidermes mélanisés ont également été préparés avec les mêmes échantillons d'ADNc. Un contrôle sans ADNc a servi de contrôle négatif d'amplification. Les cycles thermiques ont été programmés avec une étape d'incubation à 50°C pendant 2 min, suivie d'une première étape de dénaturation à 95°C pendant 10 min. Le protocole d'amplification PCR s'est poursuivi avec 40 cycles de 15 sec à 95°C suivi d'une min à 60°C.

Les niveaux d'expression ont été quantifiés selon la méthode de calcul de l'expression relative par rapport à un gène de ménage ($2^{-\Delta Ct}$), dérivée du calcul des $\Delta\Delta$Ct (Pfaffl, A new mathematical model for relative quantification in real-time RT-PCR, Nucleic Acids Res, 29 (9), 2001, 2002-2007; Livak and Schmittgen, Analysis of relative gene expression data using Real-Time Quantitative PCR and the 2-$\Delta$Ct method, Methods, 25 (4), 2001, 402-408) décrite ci-après :
La quantification relative des transcrits a été réalisée par l'utilisation d'une méthode de calcul qui consiste à comparer

la moyenne des valeurs de contrôle (Ct) obtenues pour les conditions TGF-β1 (20 ng/mL) et VD3 (100 nM) avec les conditions témoins respectives (CTL non traité et CTL Ethanol 0,1%). Ces Ct représentent le seuil de détection à partir duquel la quantité d'ADN est telle que le signal se distingue significativement du bruit de fond. Cette valeur moyenne de Ct a été normalisée par rapport à un gène de ménage, à savoir la Glycéraldehyde-3-phosphate déshydrogénase (*GAPDH*) pour les fibroblastes NHDF et la β2-microglobuline (*B2M*) pour les épidermes mélanisés reconstitués. Ainsi, la différence de niveau d'expression (RQ) a été obtenue par utilisation de la formule :

$$RQ = 2^{-(\Delta Ct\ condition\ traitée\ -\ \Delta Ct\ condition\ de\ référence)}$$

où $\Delta Ct$ = Ct (gène cible) - Ct (gène de ménage) au sein d'un même échantillon d'ADNc.

• **Préparation des cartes microfluidiques Taqman, réalisation de la PCR quantitative et analyse des Ct**

Les mélanges réactionnels pour PCR sur cartes microfluidiques TaqMan, produites à façon par Applied Biosystems, ont été préparés en suivant les instructions détaillées du guide Applied Biosystems Micro Fluidic Card Getting Started Guide. En résumé, 100 ng d'ADNc ont été ajoutés à un mélange spécifique pour PCR (Taqman Universal PCR Master Mix, 4364338, Applied Biosystems) avant d'être injectés dans la carte et dispersés par capillarité. Après avoir centrifugé la carte, celle-ci a été scellée avant la réalisation de la PCR quantitative et l'analyse avec le système 7900HT d'Applied Biosystems, à l'aide du software ABI PRISM® 7900 Sequence Détection System, SDS2.4. Les cycles seuil (Ct) ont été obtenus pour tous les gènes représentés sur les cartes et exprimés par les fibroblastes NHDF et par épidermes reconstruits mélanisés.

Les résultats ont été exportés à partir du dispositif de qPCR en temps réel en utilisant le logiciel SDS RQ Manager (v1.2.1, Applied Biosystems) et l'analyse des modifications d'expression a été réalisée à l'aide du logiciel Data Assist (V3.0., Applied Biosystems) conçu pour réaliser la quantification relative de l'expression génique en utilisant la méthode de comparaison des Ct ($\Delta\Delta$Ct) (Pfaffl, 2001 et Livak and Shmittgen, 2001) décrite dans le paragraphe ci-dessus et une combinaison d'analyses statistiques. Comme précisé plus haut, la méthode de calcul des $\Delta\Delta$Ct consiste en la comparaison des valeurs de Ct obtenues pour les conditions traitées par l'extrait avec la condition de référence (contrôle DMSO). Ces valeurs de Ct ont elles-mêmes été normalisées par rapport au gène de ménage *GAPDH* (glycéraldéhyde-3-phosphate déshydrogénase) pour les fibroblastes NHDF et *B2M* (β-2 microglobulin) pour les épidermes reconstruits mélanisés. La valeur de Ct maximale admissible utilisée comme seuil de détection a été fixée à 36 cycles.

### 7.2 Résultats

#### 7.2.1 Détermination de la concentration d'analyse de l'extrait par une étude de cytotoxicité

L'étude préalable de cytotoxicité sur les fibroblastes NHDF, sur les mélanocytes humains NHEM et sur les épidermes reconstitués a permis de définir une concentration de travail (préparé dans du DMSO) pour l'étude d'expression génique. Le solvant présent dans l'extrait a été testé en parallèle. Le SDS à 0,08% (pour NHDF) et à 0,05% (pour NHEM) a été utilisé comme contrôle positif de cytotoxicité afin de valider l'expérience. Sur la base de ces résultats (données non montrées), les concentrations suivantes ont été choisies pour la suite de l'étude : 20 µg d'extrait sec /mL pour les fibroblastes NHDF et 20 µg d'extrait sec/mL pour les épidermes mélanisés reconstitués.

#### 7.2.2 Qualification des ARNs par électrophorèse capillaire

Les différentes populations d'ARN démontrent bien la présence de pics étroits, correspondant aux ARNs ribosomiaux 18S et 28S, et un rapport équilibré entre les deux pics. L'absence de pics intermédiaires et étalés, caractéristiques de produits de dégradation des ARNs témoigne de l'intégrité des différentes populations (données non montrées). La qualité et l'intégrité des ARNs extraits étant démontrée, ceux-ci ont dès lors été utilisés pour la poursuite du protocole et engagés dans les réactions de synthèse des ADNs complémentaires.

#### 7.2.3 Effets de l'extrait sur 94 gènes cibles au sein des fibroblastes NHDF

L'extrait a été ajouté dans le milieu de culture des fibroblastes NHDF (n=3). Des contrôles traités seulement par le solvant DMSO à 1% (véhicule de l'extrait) ont également été analysés. En parallèle, le TGF-β1 (20 ng/mL) a été étudié en tant que contrôle de validation. Après 24 heures de culture des fibroblastes NHDF, les populations d'ARNs totaux ont été extraites, leur intégrité a été analysée par électrophorèse capillaire, et les différences d'expression géniques ont été analysées par qRT-PCR à l'aide de cartes TaqMan 96-puits, ciblant les fonctions clés du derme.

Ci-après, les régulations observées en lien avec ces hypothèses sont renseignées comme suit : le symbole des gènes, le nom des gènes, l'expression relative (RQ) par rapport au véhicule DMSO à 1% (RQ > 1 : augmentation) et la valeur p (p-value).

• **Structure et homéostasie de la matrice extracellulaire (MEC)**

L'extrait racinaire de *Morus alba* induit l'expression du gène *COL3A1* qui code pour la sous-unité alpha-1 du collagène de type III (X 1,5, p=0, 0172). Les collagènes fibrillaires sont, de loin, les protéines les plus abondantes dans la peau, constituant plus de 90% de son poids sec. Le collagène de type I représente 60 à 80% des collagènes du derme et de l'hypoderme alors que le collagène de type III (COL3A1) compte pour 15 à 25% et le collagène de type V pour 2 à 5%. Les collagènes de type I, III et V s'auto-assemblent en fibres plus épaisses pour former un réseau tridimensionnel dans toute l'épaisseur du derme. Ainsi, le collagène III (COL3A1) joue un rôle de structure au niveau de la MEC et donc dans la résistance et l'élasticité du derme. L'induction du gène *COL3A1* par l'extrait pourrait se révéler intéressant en vue d'une stratégie anti-âge pour permettre une diminution la dégradation du derme avec l'âge.

L'extrait racinaire de *Morus alba* induit la diminution de l'expression du gène de la métalloprotéinase-1 *MMP-1* (X 0,66, p-value = 0,0066). Les fibroblastes sécrètent des collagénases (ou MMP, métalloprotéinases) et des inhibiteurs de protéases matricielles pour dégrader la matrice extracellulaire, la renouveler et la réorganiser. La métalloprotéinase 1 (MMP1) initie le clivage des fibrilles de collagène de type I et III dans la peau.

Dans ce contexte, l'extrait se révèle intéressant pour diminuer la dégradation du derme avec l'âge. En effet, il favorise l'expression du collagène de type III tout en diminuant l'expression de MMP-1.

• **Cicatrisation cutanée**

L'extrait racinaire de *Morus alba* induit l'expression du gène ACTA2 codant pour l'actine (X 1,17, p-value = 0,04) et ayant un rôle important dans la différenciation du fibroblaste en myofibroblaste lors de la cicatrisation.

**7.2.4 Effets de l'extrait sur 94 gènes cibles au sein des épidermes reconstruits mélanisés (RHE/MEL/001)**

L'extrait à 20 µg ES/mL a été appliqué pendant 24 heures sur épidermes reconstruits. Des contrôles traités seulement par le solvant DMSO à 0,2% (véhicule de l'extrait) ont également été analysés. Le symbole des gènes, le nom des gènes, l'expression relative (RQ) par rapport au véhicule DMSO à 0,2% (RQ > 1 : augmentation - RQ < 1 : diminution) et la valeur p (p-value) sont présentés.

• **Fonction barrière de l'épiderme**

L'extrait racinaire en tant qu'inducteur de l'expression du gène *HRNR* (hornérine) avec une amplitude de 3,9 (p-value = 0,0011). L'hornérine est une protéine présente au niveau de la couche cornée et de la couche granuleuse de l'épiderme. C'est une protéine essentielle à la résistance mécanique de la couche cornée. Du fait de son effet positif sur l'expression de l'hornérine, essentielle de la couche cornée et de la couche granuleuse de l'épiderme, l'extrait présente un rôle positif dans l'intégrité et l'efficacité de la fonction barrière de l'épiderme.

• **Cicatrisation cutanée**

L'extrait racinaire de *Morus alba* induit d'une amplitude de 2,1 (p-value = 0,0289) l'expression du gène de la métalloprotéinase-1 MMP-1. Les MMPs sont des métalloprotéinases capables de cliver la plupart des composants de la MEC et de modifier, par protéolyse, bon nombre de molécules importantes pour la cicatrisation cutanée. En particulier, MMP1 joue un rôle majeur dans la ré-épithélialisation des kératinocytes. MMP1 facilite l'assemblage de la MEC, l'élongation et la migration des cellules, la réorganisation du cytosquelette d'actines, et induit l'activation de la kinase ERK (extracellular signal-regulated kinase) nécessaire à la motilité et la capacité d'invasion des cellules épithéliales. En outre, il a été montré que la protéine MMP1 est surexprimée au niveau de l'épiderme en réponse aux plaies cutanées.

La surexpression du gène MMP-1 au niveau de l'épiderme par l'extrait racinaire de *Morus alba* permet de le positionner comme actif favorisant potentiellement la cicatrisation cutanée, en particulier au niveau de la fermeture de la plaie.

• **Effet dépigmentant**

Le gène *POMC* est quant à lui réprimé (X 0,3, p-value = 0,0175). Ce dernier code pour la proopiomélanocortine, une protéine précurseur de plusieurs hormones pituitaires dont l'alpha-MSH (alpha-melanocyte-stimulating hormone)

qui est connue pour stimuler la mélanogenèse. En diminuant l'expression de *POMC,* l'extrait racinaire de *Morus alba* pourrait donc avoir un effet dépigmentant de l'épiderme.

### 7.3 Conclusion

En synthèse, l'extrait racinaire de *Morus alba* se positionne clairement par rapport à une revendication cosmétique dans une optique anti-âge, c'est-à-dire prévenir ou retarder l'apparition des effets du vieillissement, liée à une réduction de la dégradation du derme associé à un rôle positif dans l'intégrité et l'efficacité de la fonction barrière de l'épiderme.
De plus, l'extrait racinaire de *Morus alba* pourrait avoir un effet dépigmentant de l'épiderme.
Enfin, l'extrait racinaire de *Morus alba* se positionne éventuellement comme un actif favorisant la cicatrisation cutanée, en particulier dans le cas de la fermeture d'une plaie.

## Revendications

**1.** Extrait racinaire de plante du genre *Morus,* ladite plante étant choisie dans le groupe constitué par *Morus alba* et *Morus nigra,* **caractérisé en ce que** :

- il comprend au moins 2 % en poids de moracénine B, de préférence au moins 2,3 % et plus préférentiellement au moins 4 %, exprimé par rapport au poids total de l'extrait sec,
- il comprend de la moracénine A, présente en une quantité d'au moins 0,05 % en poids d'équivalent moracénine B, de préférence au moins 0,5 % et plus préférentiellement au moins 2 %, exprimée par rapport au poids total de l'extrait sec,
- il comprend de la kuwanone C, présente en une quantité d'au moins 0,1 % en poids d'équivalent moracénine B, de préférence au moins 0,5 % et plus préférentiellement au moins 1 %, exprimée par rapport au poids total de l'extrait sec, et
- il comprend au moins un des deux composés suivants :

  - de la wittiorumine F, présente en une quantité d'au moins 0,1 % en poids d'équivalent moracénine B, de préférence au moins 0,4 %, et plus préférentiellement au moins 0,5 %, exprimée par rapport au poids total de l'extrait sec,
  - du mulberrofurane T, présent en une quantité d'au moins 0,1 % en poids d'équivalent moracénine B, de préférence au moins 0,3 %, et plus préférentiellement au moins 0,4 %, exprimée par rapport au poids total de l'extrait sec.

**2.** Extrait racinaire selon la revendication 1, **caractérisé en ce que** :

- il comprend au moins 2,3 % en poids de moracénine B, exprimé par rapport au poids total de l'extrait sec,
- il comprend de la moracénine A, présente en une quantité d'au moins 0,5 % en poids d'équivalent moracénine B, exprimée par rapport au poids total de l'extrait sec,
- il comprend de la kuwanone C, présente en une quantité d'au moins 0,5 % en poids d'équivalent moracénine B, exprimée par rapport au poids total de l'extrait sec, et
- il comprend du mulberrofurane T, présent en une quantité d'au moins 0,1 % en poids d'équivalent moracénine B, de préférence au moins 0,3 %, et plus préférentiellement au moins 0,4 %, exprimée par rapport au poids total de l'extrait sec.

**3.** Extrait racinaire selon l'une des revendications 1 ou 2, **caractérisé en ce que** :

- il comprend de la moracénine A, présente en une quantité d'au moins 1 %, de préférence au moins 1,5% en poids d'équivalent moracénine B, exprimée par rapport au poids total de l'extrait sec,
- il comprend de la kuwanone C, présente en une quantité d'au moins 1 % en poids d'équivalent moracénine B, exprimée par rapport au poids total de l'extrait sec.

**4.** Extrait racinaire selon l'une des revendications 1 à 3, **caractérisé en ce que** :

- il comprend du mulberrofurane T, présent en une quantité d'au moins 0,3%, de préférence au moins 0,4 % en poids d'équivalent moracénine B, exprimée par rapport au poids total de l'extrait sec.

**5.** Extrait racinaire selon l'une des revendications 1 à 3, **caractérisé en ce que** :

- il comprend du mulberrofurane T, présent en une quantité d'au moins 0,1 % en poids d'équivalent moracénine B, de préférence au moins 0,3 %, et plus préférentiellement au moins 0,4 %, exprimée par rapport au poids total de l'extrait sec et
- il comprend de la wittiorumine F, présente en une quantité d'au moins 0,1 % en poids d'équivalent moracénine B, de préférence au moins 0,4 %, et plus préférentiellement au moins 0,5 %, exprimée par rapport au poids total de l'extrait sec.

**6.** Procédé de préparation d'un extrait racinaire de plantes du genre *Morus* selon l'une des revendications 1 à 5, comprenant :

a) une étape de culture de plantes du genre *Morus* en conditions horssol, de préférence en aéroponie,
b) optionnellement une étape de stimulation des racines des plantes,
c) une étape d'extraction solide/liquide des racines optionnellement stimulées lors de l'étape b), et
d) la récupération de l'extrait obtenu lors de l'étape c).

**7.** Procédé de préparation d'un extrait racinaire de plantes du genre *Morus* selon la revendication 6, comprenant une étape de stimulation des racines des plantes.

**8.** Procédé selon l'une des revendications 6 ou 7, dans lequel l'étape c) comprend la coupe, le séchage et le broyage desdites racines, suivis de la macération des racines broyées dans un solvant, ledit solvant étant choisi parmi les alcools, les glycols et les solvants eutectiques, et étant utilisé pur ou sous la forme d'une solution aqueuse d'alcool, de glycol ou de solvant eutectique.

**9.** Composition cosmétique comprenant, en tant qu'agent actif, en une quantité comprise entre 0,01 et 10 % en poids par rapport au poids total de la composition, au moins un extrait selon l'une des revendications 1 à 5, ou un extrait obtenu par un procédé selon l'une des revendications 6 à 8, et au moins un excipient cosmétiquement acceptable.

**10.** Extrait selon l'une des revendications 1 à 5, extrait obtenu par un procédé selon l'une des revendications 6 à 8, ou composition cosmétique selon la revendication 9, pour prévenir ou retarder l'apparition des effets du vieillissement de la peau et/ou pour avoir un effet blanchiment de la peau.

**11.** Composition pharmaceutique comprenant, en tant qu'agent actif, en une quantité comprise entre 0,01 et 10 % en poids par rapport au poids total de la composition, au moins un extrait selon l'une des revendications 1 à 5, ou un extrait obtenu par un procédé selon l'une des revendications 6 à 8, et au moins un excipient pharmaceutiquement acceptable.

**12.** Extrait selon l'une des revendications 1 à 5, extrait obtenu par un procédé selon l'une des revendications 6 à 8, ou composition pharmaceutique selon la revendication 11 pour son utilisation en tant que médicament pour favoriser la cicatrisation cutanée, en particulier dans le cas de la fermeture d'une plaie.

**13.** Utilisation d'un extrait selon l'une des revendications 1 à 5, ou d'un extrait obtenu par un procédé selon l'une des revendications 6 à 8, pour la préparation d'une composition cosmétique comprenant en tant qu'agent actif une quantité comprise entre 0,01 et 10% en poids par rapport au poids total de la composition dudit extrait, visant à prévenir ou retarder l'apparition des effets du vieillissement de la peau et/ou visant un effet blanchiment de la peau.

## Patentansprüche

**1.** Wurzelextrakt einer Pflanze der Gattung *Morus,* wobei die Pflanze aus der Gruppe ausgewählt ist, die aus *Morus alba* und *Morus nigra* besteht, **dadurch gekennzeichnet, dass**:

- er wenigstens 2 Gew.-% Moracenin B, vorzugsweise wenigstens 2,3 Gew.-% und besonders bevorzugt wenigstens 4 Gew.-% umfasst, bezogen auf das Gesamtgewicht des Trockenextrakts;
- er Moracenin A umfasst, das in einer Menge von wenigstens 0,05 Gew.-% Moracenin-B-Äquivalent, vorzugsweise wenigstens 0,5 Gew.-% und besonders bevorzugt wenigstens 2 Gew.-% vorhanden ist, bezogen auf das Gesamtgewicht des Trockenextrakts;

• er Kuwanon C umfasst, das in einer Menge von wenigstens 0,1 Gew.-% Moracenin-B-Äquivalent, vorzugsweise wenigstens 0,5 Gew.-% und besonders bevorzugt wenigstens 1 Gew.-% vorhanden ist, bezogen auf das Gesamtgewicht des Trockenextrakts; und
• er wenigstens eine der beiden folgenden Verbindungen umfasst:

- Wittiorumin F, das in einer Menge von wenigstens 0,1 Gew.-% Moracenin-B-Äquivalent, vorzugsweise wenigstens 0,4 Gew.-% und besonders bevorzugt wenigstens 0,5 Gew.-% vorhanden ist, bezogen auf das Gesamtgewicht des Trockenextrakts;
- Mulberrofuran T, das in einer Menge von wenigstens 0,1 Gew.-% Moracenin-B-Äquivalent, vorzugsweise wenigstens 0,3 Gew.-% und besonders bevorzugt wenigstens 0,4 Gew.-% vorhanden ist, bezogen auf das Gesamtgewicht des Trockenextrakts.

**2.** Wurzelextrakt gemäß Anspruch 1, **dadurch gekennzeichnet, dass**:

• er wenigstens 2,3 Gew.-% Moracenin B umfasst, bezogen auf das Gesamtgewicht des Trockenextrakts;
• er Moracenin A umfasst, das in einer Menge von wenigstens 0,5 Gew.-% Moracenin-B-Äquivalent vorhanden ist, bezogen auf das Gesamtgewicht des Trockenextrakts;
• er Kuwanon C umfasst, das in einer Menge von wenigstens 0,5 Gew.-% Moracenin-B-Äquivalent vorhanden ist, bezogen auf das Gesamtgewicht des Trockenextrakts; und
• er Mulberrofuran T umfasst, das in einer Menge von wenigstens 0,1 Gew.-% Moracenin-B-Äquivalent, vorzugsweise wenigstens 0,3 Gew.-% und besonders bevorzugt wenigstens 0,4 Gew.-% vorhanden ist, bezogen auf das Gesamtgewicht des Trockenextrakts.

**3.** Wurzelextrakt gemäß einem der Ansprüche 1 oder 2, **dadurch gekennzeichnet, dass**:

• er Moracenin A umfasst, das in einer Menge von wenigstens 1 Gew.-%, vorzugsweise wenigstens 1,5 Gew.-%, Moracenin-B-Äquivalent vorhanden ist, bezogen auf das Gesamtgewicht des Trockenextrakts;
• er Kuwanon C umfasst, das in einer Menge von wenigstens 1 Gew.-% Moracenin-B-Äquivalent vorhanden ist, bezogen auf das Gesamtgewicht des Trockenextrakts.

**4.** Wurzelextrakt gemäß einem der Ansprüche 1 bis 3, **dadurch gekennzeichnet, dass**:

• er Mulberrofuran T umfasst, das in einer Menge von wenigstens 0,3 Gew.-%, vorzugsweise wenigstens 0,4 Gew.-%, Moracenin-B-Äquivalent vorhanden ist, bezogen auf das Gesamtgewicht des Trockenextrakts.

**5.** Wurzelextrakt gemäß einem der Ansprüche 1 bis 3, **dadurch gekennzeichnet, dass**:

• er Mulberrofuran T umfasst, das in einer Menge von wenigstens 0,1 Gew.-% Moracenin-B-Äquivalent, vorzugsweise wenigstens 0,3 Gew.-% und besonders bevorzugt wenigstens 0,4 Gew.-% vorhanden ist, bezogen auf das Gesamtgewicht des Trockenextrakts; und
• er Wittiorumin F umfasst, das in einer Menge von wenigstens 0,1 Gew.-% Moracenin-B-Äquivalent, vorzugsweise wenigstens 0,4 Gew.-% und besonders bevorzugt wenigstens 0,5 Gew.-% vorhanden ist, bezogen auf das Gesamtgewicht des Trockenextrakts.

**6.** Verfahren zur Herstellung eines Wurzelextrakts von Pflanzen der Gattung *Morus* gemäß einem der Ansprüche 1 bis 5, umfassend:

a) einen Schritt der Kultur von Pflanzen der Gattung *Morus* unter Hydroponikbedingungen, vorzugsweise durch Aeroponik;
b) gegebenenfalls einen Schritt der Stimulation der Wurzeln der Pflanzen;
c) einen Schritt der Fest/Flüssig-Extraktion der in Schritt b) gegebenenfalls stimulierten Wurzeln; und
d) die Gewinnung des in Schritt c) erhaltenen Extrakts.

**7.** Verfahren zur Herstellung eines Wurzelextrakts von Pflanzen der Gattung *Morus* gemäß Anspruch 6, umfassend einen Schritt der Stimulation der Wurzeln der Pflanzen.

**8.** Verfahren gemäß einem der Ansprüche 6 oder 7, wobei Schritt c) das Abschneiden, Trocknen und Mahlen der Wurzeln mit anschließendem Auslaugen der gemahlenen Wurzeln in einem Lösungsmittel umfasst, wobei das

Lösungsmittel aus Alkoholen, Glycolen und eutektischen Lösungsmitteln ausgewählt ist und unverdünnt oder in Form einer wässrigen Lösung von Alkohol, von Glycol oder von eutektischem Lösungsmittel verwendet wird.

**9.** Kosmetikzusammensetzung, umfassend als Wirkstoff in einer Menge zwischen 0,01 und 10 Gew.-%, bezogen auf das Gesamtgewicht der Zusammensetzung, wenigstens einen Extrakt gemäß einem der Ansprüche 1 bis 5 oder einen Extrakt, der nach einem Verfahren gemäß einem der Ansprüche 6 bis 8 erhalten wurde, und wenigstens einen kosmetisch annehmbaren Hilfsstoff.

**10.** Extrakt gemäß einem der Ansprüche 1 bis 5, Extrakt, der nach einem Verfahren gemäß einem der Ansprüche 6 bis 8 erhalten wurde, oder Kosmetikzusammensetzung gemäß Anspruch 9 zur Verhinderung oder Verzögerung des Auftretens von Wirkungen der Alterung der Haut und/oder zum Bewirken einer Bleichung der Haut.

**11.** Pharmazeutische Zusammensetzung, umfassend als Wirkstoff in einer Menge zwischen 0,01 und 10 Gew.-%, bezogen auf das Gesamtgewicht der Zusammensetzung, wenigstens einen Extrakt gemäß einem der Ansprüche 1 bis 5 oder einen Extrakt, der nach einem Verfahren gemäß einem der Ansprüche 6 bis 8 erhalten wurde, und wenigstens einen pharmazeutisch annehmbaren Hilfsstoff.

**12.** Extrakt gemäß einem der Ansprüche 1 bis 5, Extrakt, der nach einem Verfahren gemäß einem der Ansprüche 6 bis 8 erhalten wurde, oder pharmazeutische Zusammensetzung gemäß Anspruch 11 zur Verwendung als Medikament zur Begünstigung der Wundheilung der Haut, insbesondere im Fall eines Wundverschlusses.

**13.** Verwendung eines Extrakts gemäß einem der Ansprüche 1 bis 5 oder eines Extrakts, der nach einem Verfahren gemäß einem der Ansprüche 6 bis 8 erhalten wurde, zur Herstellung einer Kosmetikzusammensetzung, die als Wirkstoff eine Menge zwischen 0,01 und 10 Gew.-%, bezogen auf das Gesamtgewicht der Zusammensetzung, des Extrakts umfasst und die zur Verhinderung oder Verzögerung des Auftretens von Wirkungen der Alterung der Haut und/oder zum Bewirken einer Bleichung der Haut bestimmt ist.

## Claims

**1.** Root extract of plants of the genus *Morus,* wherein said plant is chosen from the group consisting of *Morus alba* and *Morus nigra,* **characterized in that**:

- it comprises at least 2% by weight of moracenin B, preferably at least 2.3% and more preferably at least 4%, expressed relative to the total weight of the dry extract,
- it comprises moracenin A, present in an amount of at least 0.05% by weight of moracenin B equivalent, preferably at least 0.5% and more preferably at least 2%, expressed relative to the total weight dry extract,
- it comprises kuwanone C, present in an amount of at least 0.1% by weight of moracenin B equivalent, preferably at least 0.5% and more preferably at least 1%, expressed relative to the total weight of the dry extract, and
- it comprises at least one of the following two compounds:

  - wittiorumine F, present in an amount of at least 0.1% by weight of moracenin B equivalent, preferably at least 0.4%, and more preferably at least 0.5%, expressed relative to the total weight dry extract,
  - mulberrofuran T, present in an amount of at least 0.1% by weight, preferably at least 0.3%, and more preferably at least 0.4%, expressed relative to the total weight dry extract.

**2.** Root extract according to claim 1, **characterized in that**:

- it includes at least 2.3% by weight of moracenin B, expressed relative to the total weight of the dry extract,
- it includes moracenin A, present in an amount of at least 0.5% by weight of moracenin B equivalent, expressed in relation to the total weight of the dry extract,
- it includes kuwanone C, present in an amount of at least 0.5% by weight of moracenin B equivalent, expressed in relation to the total weight of the dry extract, and
- it comprises mulberrofuran T, present in an amount of at least 0.1% by weight of moracenin B equivalent, preferably at least 0.3%, and more preferably at least 0.4%, expressed relative to the total weight of dry extract.

**3.** Root extract according to any of claims 1 or 2, **characterized in that**:

• it comprises moracenin A, present in an amount of at least 1%, preferably at least 1.5% by weight of moracenin B equivalent, expressed relative to the total weight of the dry extract,
• it includes kuwanone C, present in an amount of at least 1% by weight of moracenin B equivalent, expressed in relation to the total weight of the dry extract.

**4.** Root extract according to any of claims 1 to 3, **characterized in that** it comprises mulberrofuran T, present in an amount of at least 0.3%, preferably at least 0.4% by weight of moracenin B equivalent, expressed relative to the total weight of the dry extract.

**5.** Root extract to any of claims 1 to 3, **characterized in that**:

• it comprises mulberrofuran T, present in an amount of at least 0.1% by weight of moracenin B equivalent, preferably at least 0.3%, and more preferably at least 0.4%, expressed relative to the total weight of dry extract and
• it comprises wittiorumine F, present in an amount of at least 0.1% by weight of moracenin B equivalent, preferably at least 0.4%, and more preferably at least 0.5%, expressed relative to the total weight of the dry extract.

**6.** Process for the preparation of a root extract of plants of the genus *Morus* according to any of claims 1 to 5, comprising

a) a step of cultivating plants of the *Morus* genus in above-ground conditions, preferably in aeroponics,
b) optionally a stage of stimulation of the roots of the plants,
c) a stage of solid/liquid extraction of the roots optionally stimulated during stage b), and
d) the recovery of the extract obtained during stage c ).

**7.** Process for the preparation of a root extract of plants of the genus *Morus* according to claim 6, comprising a step of stimulating the roots of the plants.

**8.** Process according to any of claim 6 or 7, wherein step c) comprises the cutting, drying and grinding of said roots, followed by maceration of the ground roots in a solvent, said solvent being chosen from alcohols, glycols and eutectic solvents, and being used pure or in the form of an aqueous solution of alcohol, glycol or eutectic solvent.

**9.** Cosmetic composition comprising, as active agent, in an amount of between 0.01 and 10% by weight relative to the total weight of the composition, at least one extract according to any of claims 1 to 5, or an extract obtained by a process according to any of claims 6 to 8, and at least one cosmetically acceptable excipient.

**10.** Extract according to any of claims 1 to 5, extract obtained by a process according to any of claims 6 to 8, or cosmetic composition according to claim 9, for preventing or delaying the appearance of the effects of skin aging and/or to have a whitening effect on the skin.

**11.** Pharmaceutical composition comprising, as active agent, in an amount of between 0.01 and 10% by weight relative to the total weight of the composition, at least one extract according to any of claims 1 to 5, or an extract obtained by a process according to any of claims 6 to 8, and at least one pharmaceutically acceptable excipient.

**12.** Extract according to any of claims 1 to 5, extract obtained by a process according to any of claims 6 to 8, or pharmaceutical composition according to claim 11 for its use as a medicament for promoting skin healing, in particular in the case of wound closure.

**13.** Use of an extract according to any of claims 1 to 5, or of an extract obtained by a process according to any of claims 6 to 8, for the preparation of a cosmetic composition comprising, as active agent in an amount of between 0.01 and 10% by weight relative to the total weight of the composition of said extract, aimed at preventing or delaying the appearance of the effects of aging of the skin and/or aiming at a whitening effect on the skin.

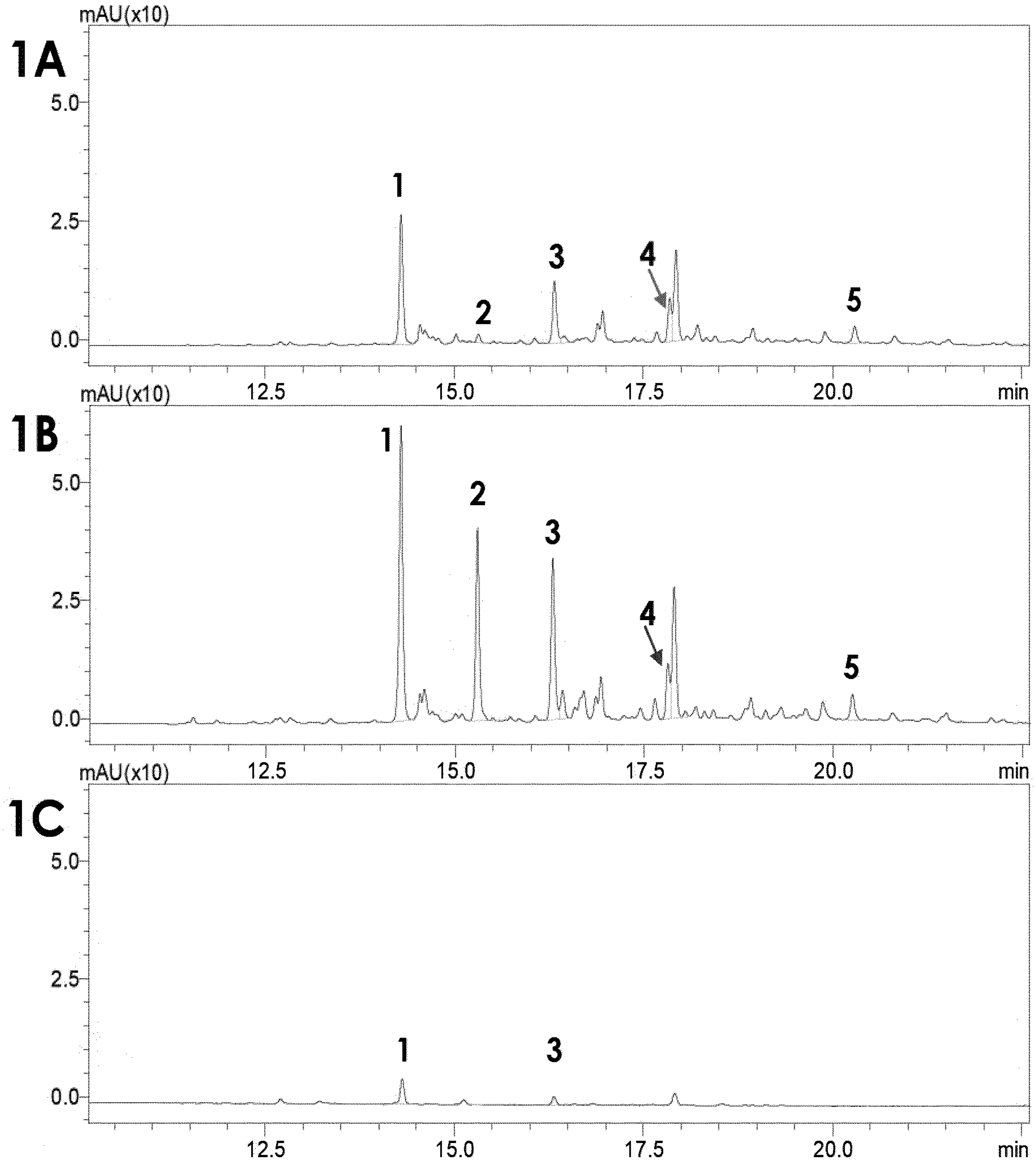


Fig. 1A, 1B, 1C

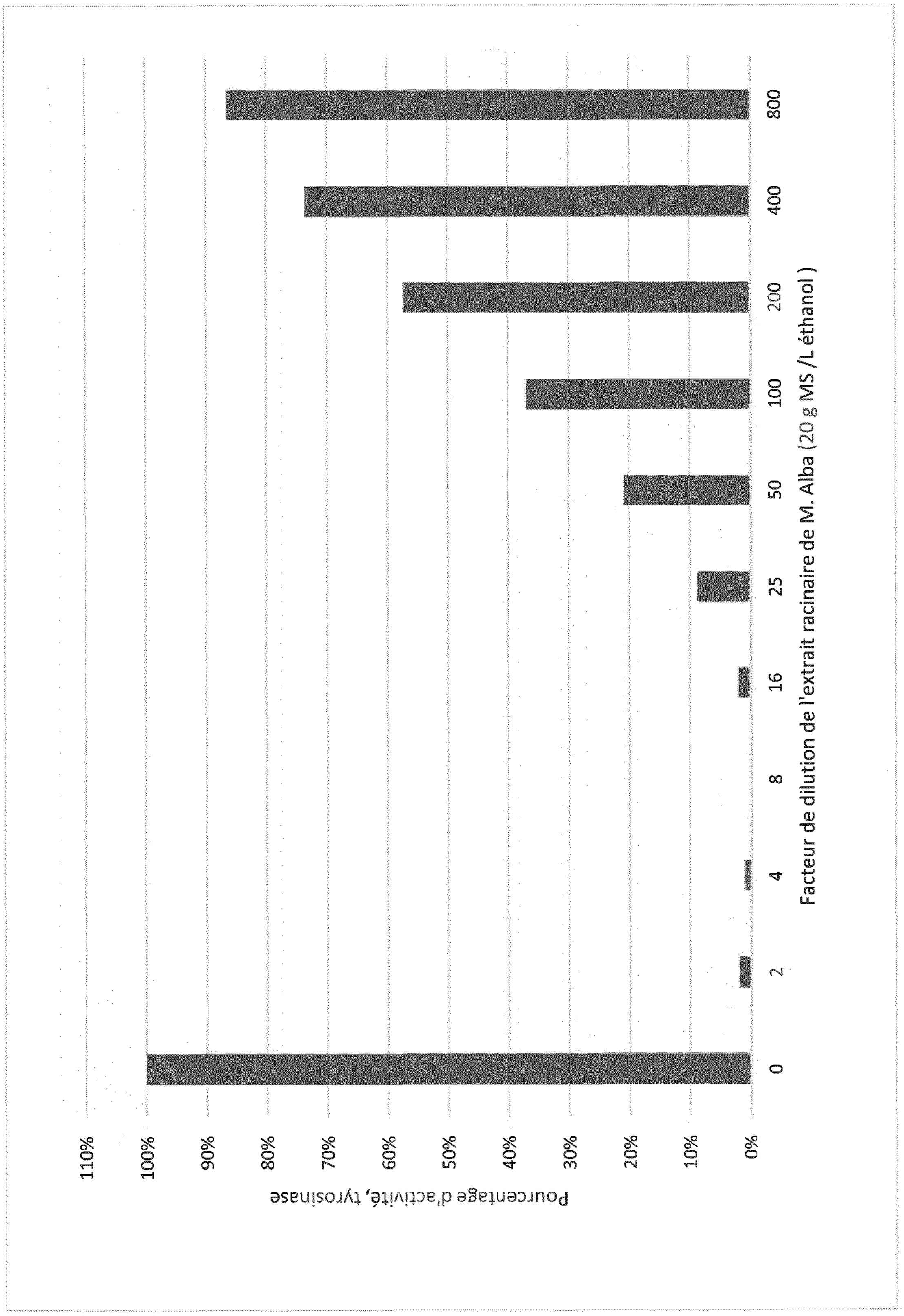
Pourcentage d'activité, tyrosinase
110%
100%
90%
80%
70%
60%
50%
40%
30%
20%
10%
0%
0
2
4
8
16
25
50
100
200
400
800
Facteur de dilution de l'extrait racinaire de M. Alba (20 g MS /L éthanol )

Fig. 2

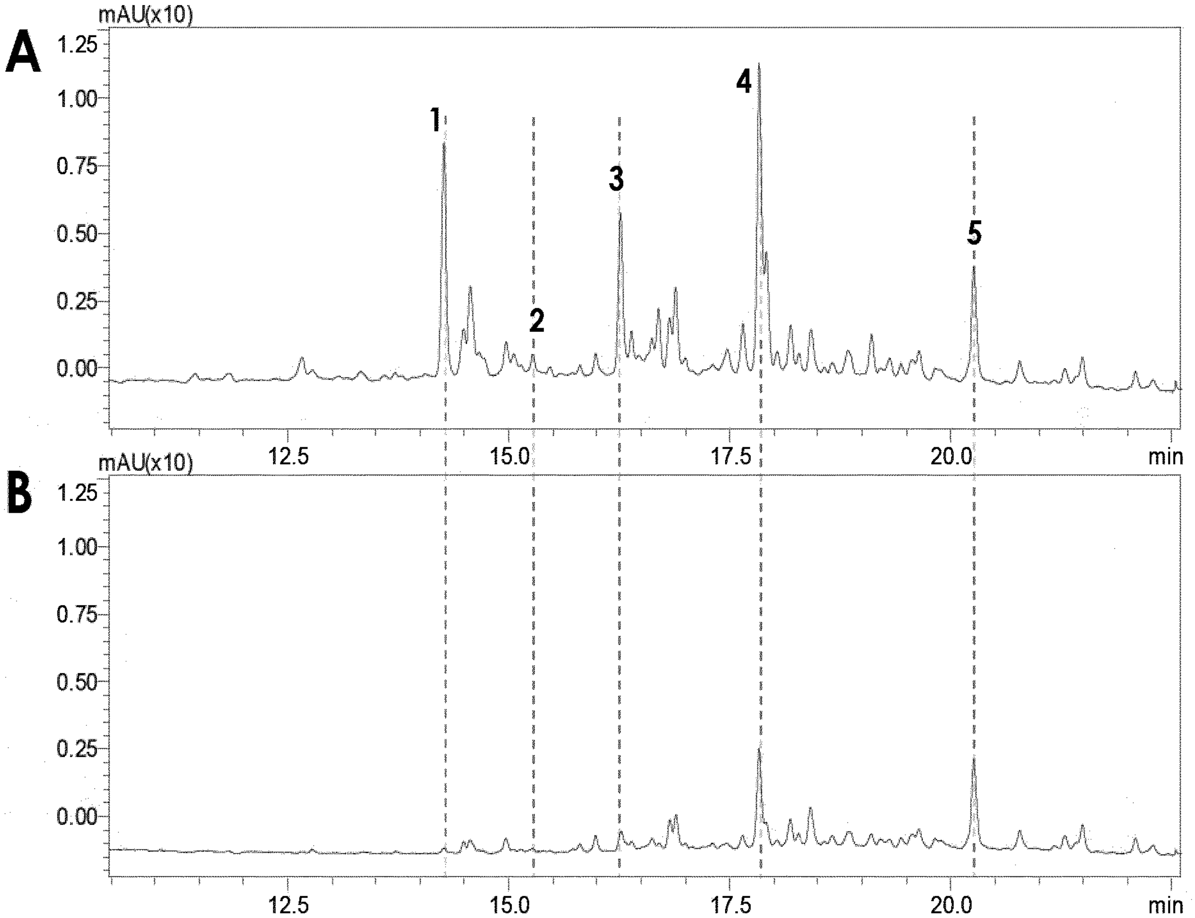
A
mAU(x10)
1.25
1.00
0.75
0.50
0.25
0.00
1
2
3
4
5
12.5
15.0
17.5
20.0
min
B
mAU(x10)
1.25
1.00
0.75
0.50
0.25
0.00
12.5
15.0
17.5
20.0
min

**Fig. 3A, 3B**

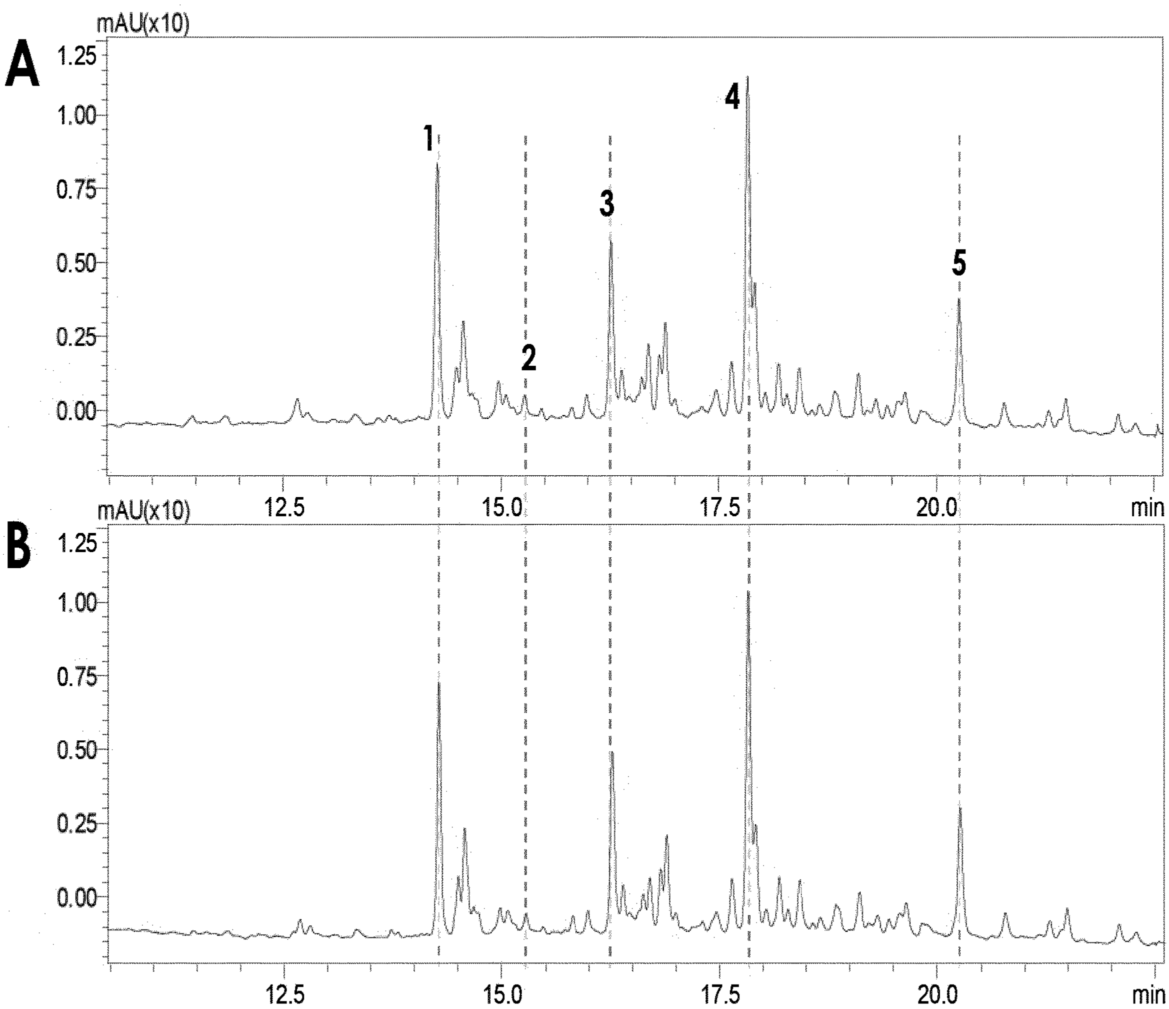


**Fig. 4A, 4B**

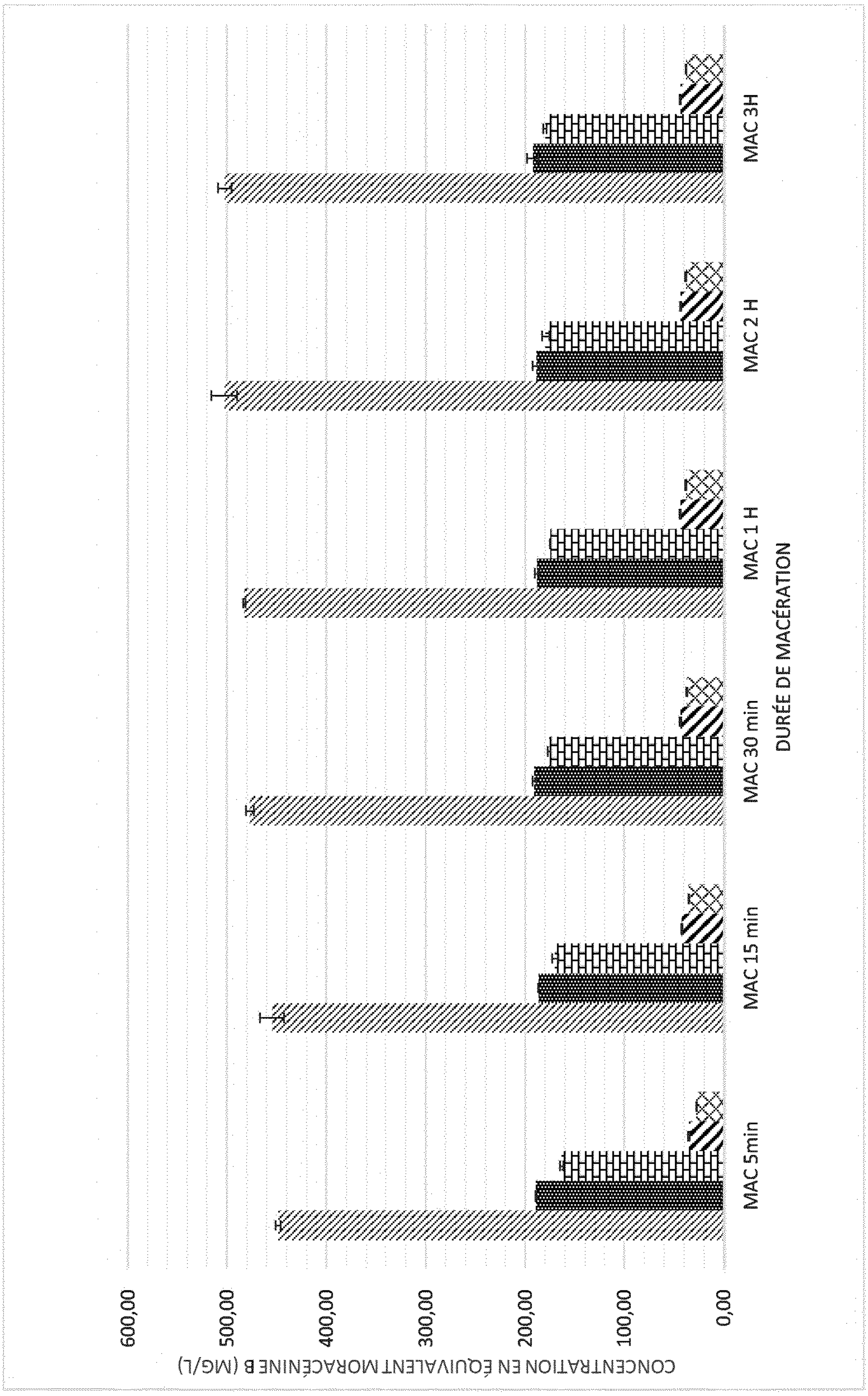

CONCENTRATION EN ÉQUIVALENT MORACÉNINE B (MG/L)
600,00
500,00
400,00
300,00
200,00
100,00
0,00
MAC 5min
MAC 15 min
MAC 30 min
MAC 1 H
MAC 2 H
MAC 3H
DURÉE DE MACÉRATION


Fig. 5

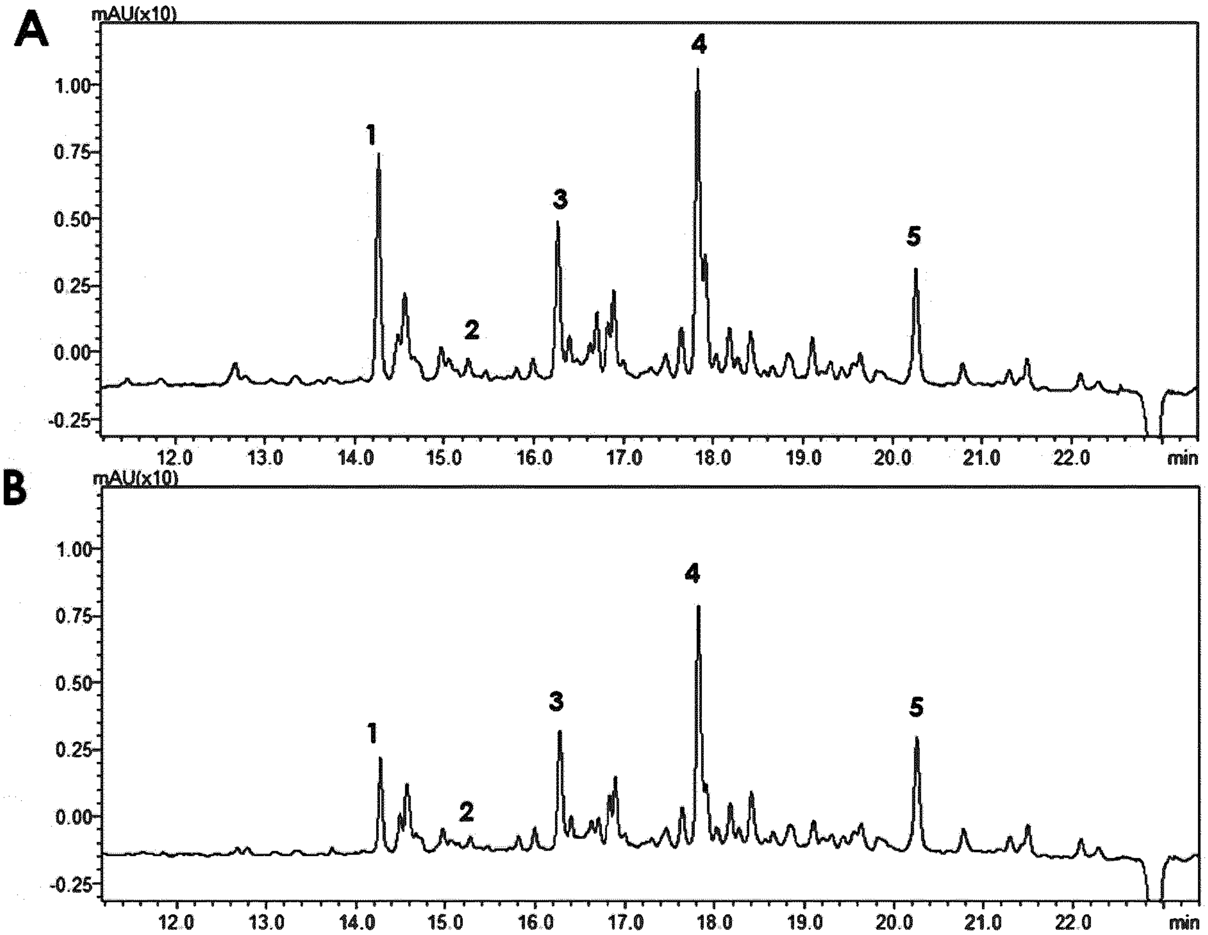
A
mAU(x10)
1.00
0.75
0.50
0.25
0.00
-0.25
12.0
13.0
14.0
15.0
16.0
17.0
18.0
19.0
20.0
21.0
22.0
min
1
2
3
4
5
B
mAU(x10)
1.00
0.75
0.50
0.25
0.00
-0.25
12.0
13.0
14.0
15.0
16.0
17.0
18.0
19.0
20.0
21.0
22.0
min
1
2
3
4
5

**Fig. 6A, 6B**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2013181296 A
- FR 1670545
- WO 0133942 A
- WO 2004101479 A


### Littérature non-brevet citée dans la description


- **YANG Y. et al.** The latest review on the polyphenols and their bioactivities of Chinese Morus plants. *J Asian Nat Prod Res.,* 2014, vol. 16 (6), 690-702
- **ZHENG ZP et al.** Tyrosinase inhibitory constituents from the roots of Morus nigra: a structure-activity relationship study. *J Agric Food Chem.,* 12 Mai 2010, vol. 58 (9), 5368-73
- **KYO BIN KANG et al.** Prédiction of tyrosinase inhibitory activities of Morus alba root bark extracts from HPLC fingerprints. *Microchemical Journal,* 2013, vol. 110, 731-738
- **JEONG JY et al.** Characterization of Melanogenesis Inhibitory Constituents of Morus alba Leaves and Optimization of Extraction Conditions Using Response Surface MethodologyMolecules. *Molecules,* 14 Mai 2015, vol. 20 (5), 8730-41
- **TAN YX et al.** Wittiorumins A - F, antioxidant diels-alder-type adducts from Morus wittiorum. *Planta Med.,* Février 2009, vol. 75 (3), 249-55
- **KANG J. et al.** *Planta Med.,* Janvier 2006, vol. 72 (1), 52-9
- **PFAFFL.** A new mathematical model for relative quantification in real-time RT-PCR. *Nucleic Acids Res,* 2001, vol. 29 (9), 2002-2007
- **LIVAK ; SCHMITTGEN.** Analysis of relative gene expression data using Real-Time Quantitative PCR and the 2-ΔCt method. *Methods,* 2001, vol. 25 (4), 402-408